(19) Europäisches Patentamt
European Patent Office
Office européen des brevets

(11) EP 1 862 188 B1

(12) **EUROPEAN PATENT SPECIFICATION**

(45) Date of publication and mention of the grant of the patent:
**28.02.2018 Bulletin 2018/09**

(51) Int Cl.:
***A61L 27/18*** *(2006.01)* ***A61L 27/56*** *(2006.01)*
***A61L 27/58*** *(2006.01)* ***A61L 27/38*** *(2006.01)*
***A61L 31/14*** *(2006.01)* ***A61L 31/06*** *(2006.01)*

(21) Application number: **06011457.6**

(22) Date of filing: **02.06.2006**

(54) **Porous membrane comprising a biocompatible block-copolymer**

Poröse Membrane mit biokompatiblem Blockcopolymer

Membrane poreuse comprenant un copolymère à blocs

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**05.12.2007 Bulletin 2007/49**

(73) Proprietor: **Eidgenössische Technische Hochschule Zürich**
**8092 Zürich (CH)**

(72) Inventor: **Neuenschwander, Peter**
**5400 Baden (CH)**

(74) Representative: **Schaad, Balass, Menzl & Partner AG**
**Dufourstrasse 101**
**Postfach**
**8034 Zürich (CH)**

(56) References cited:
**EP-A- 1 452 189**
**EP-A- 1 452 190**
**EP-A- 1 498 147**
**EP-A1- 0 696 605**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 862 188 B1

## Description

The present invention relates to a membrane comprising a biocompatible block-copolymer obtainable by a polycondensation of at least two block units. A method for preparing said membrane is also provided.

Despite the vast number of polymers available nowadays there are only a few which are employed in the biomedical field. This holds especially true with respect to implants. The reasons for this situation are basically biocompatibility, mechanical properties, such as stiffness and elasticity, sterilizability, degradability of these polymers and a steadily growing number of administrative regulations in different countries which have to be met when using such polymers for medical purposes.

The process of formation of membranes is quite complex and difficult to control. In many cases, the resulting membranes are hardly or not permeable at all.

EP 0 196 486 discloses a biocompatible block copolymer which can be used as medical implant. This block copolymer has a crystalline and an amorphous component. The degradability of these block copolymers is, however, not fast enough for all applications.

EP 1 498 147 describes a biocompatible block copolymer with a controllable degradability. The block copolymer may also be employed in medical implants.

EP 1 452 189 discloses a shaped article comprising polyglycolic acid as a dimensionally stable carrier material. The surface of said carrier is coated with a biocompatible and degradable block copolymer. The shaped article may be used as an implant.

EP 1 452 190 describes an implant with a coating comprising a biocompatible block copolymer and on top of the block copolymer a polylysine layer.

US 2005/0155926 discloses a terpolymer made of tetrafluoroethylene, hexafluoropropylene and vinylidene fluoride. A classical method for producing a membrane comprising said terpolymer is also provided.

Membranes have been prepared by a variety of methods. A classical method is known as "non-solvent induced phase separation" (NIPS). In this method the polymer is dissolved in a solvent. A film of this solution is disposed on a carrier. The film is then contacted with a fluid which is a non-solvent for the polymer, but which is miscible with the solvent to induce phase inversion of the film.

Yet another method that has been used to prepare membranes is called "diffusion induced phase separation. The polymer solution is brought into contact with a coagulation bath. The solvent diffuses outwards into the coagulation bath while the non-solvent diffuses into the cast film. The exchange of solvent and non-solvent yields a solution which becomes thermodynamically unstable resulting in the separation of the components. A flat membrane is obtained.

The term medicine or medical as used herein means both human and veterinary medicine.

Membrane as used herein means a typically planar, porous structure. However, the membrane can also have different shapes, e.g. a tubular shape or be a hollow fibre.

The term "pore" as used herein means a minute space in a material. The minute space has a highly complex and irregular form.

Mesh as used herein means a typically planar network comprising fibers that are interconnected in regular or irregular manner.

The problem of the present invention is to provide membranes comprising a block copolymer which are biodegradable, have very good sterilizability and excellent mechanical properties, e.g. stiffness and elasticity and which have a uniform porous structure.

The problem is solved by a membrane according to claim 1. Further preferred embodiments are the subject-matter of dependent claims 2 to 14.

The membranes of the present invention comprise a block copolymer which is highly biocompatible and also biodegradable. The degradability can be precisely controlled by minor changes in the chemical composition of the membrane. The base material of the membrane is a pure polymer. No further additives such as stabilizers, antioxidants or plastifiers, which could adversely affect the excellent biocompatibility, are needed. Further advantageous properties are their elasticity while still maintaining excellent mechanical stiffness. The mechanical properties strongly depend on the crystalline and the non-crystalline compound.

Membranes according to the present invention have porous structure. Pores are present throughout the entire membrane and they are regularly distributed in the membrane resulting in a regularly structured membrane. The membranes are considered as symmetric, since the interfaces on both sides show the same structures, with only minimal differences compared with the structure of the inside. In addition, the average pore size varies only within a limited range. The porosity and the pore size of the membrane of the present invention can be varied according to needs of the intended use making the membranes a very versatile tool. Porosity can on one hand be controlled by the concentration of the block copolymer solution and on the other hand by the choice of the solvent. An increasing concentration leads to a decrease of the pore size.

It is possible that liquids and (macro-)molecules diffuse into or even pass through the membrane of the present

invention. This permeability, obeying Fick's equation, is a great advantage, if the membrane is used in biological systems. There it is very important that artificial membranes allow the exchange of gases, e.g. oxygen, liquids and compounds, e.g. nutrients for and waste of cells. If such exchange is hampered or not possible at all, cells which are in close contact with such membranes may die. Membranes according to present invention are permeable or semipermeable.

The permeability depends on the size of the pores. Membranes of the present invention have pores in a size range of 0,2 to 20.0 μm, preferably in the size range of 0,2 to 10,0 μm. The permeability for such membranes (e.g. Fig. 6) defined as

$$P = \frac{\text{(amount of permeant) (membrane thickness)}}{\text{(area) (time) (driving force gradient across membrane)}}$$

The permeability of membranes according to the present for water at 25°C is in the order of 1 x $10^{-6}$ kg/m•sec•Pa. The permeability of membranes according to the present invention is in the range of 0.1 x $10^{-6}$ to 5 x $10^{-6}$ kg/m•sec•Pa.

The membranes of the invention are extremely biocompatible in vitro cell cultures with macrophages and fibroblasts owing to the observation of cell adhesion, cell growth, cell vitality and cell activation, and of the production of extracellular proteins and cytokines.

The mechanical properties and the degradability may be changed almost independently from each other. This combination of membrane properties is unique. Since membranes performing a separation process usually are also mechanically stressed, their mechanical characteristics are also of importance. A typical membrane (as e.g. Fig. 6) has a mechanical performance in the range of the values indicated in the following table:

Table 1

| Mechanical properties | Mean ± SD |
|---|---|
| Tensile strength (km) | 0.15 ± 0.04 |
| Elongation to break (%) | 115.59 ± 46.31 |
| Modulus of elasticity (km) | 0.42 ± 0.05 |

Membranes according to the present invention comprise a biocompatible block copolymer. Suitable biocompatible block copolymers have been described in EP 0 696 605 and EP 1 498 147.

This block copolymer has at least two block units obtainable by linear polycondensation in the presence of diisocyanate, diacid halide or phosgene of a first block unit being an α,ω-dihydroxy-polyester (IV) with a second block unit selected from the group consisting of a diol (I), an α,ω-dihydroxy-polyester (II), an α,ω-dihydroxy-polyether (III) and the same α,ω-dihydroxy-polyester (IV). The diol (I) is obtainable by transesterification of poly-[(R)-(3)-hydroxybutyric acid] or copolymers thereof with 3-hydroxyvaleric acid with ethylene glycol.

The α,ω-dihydroxy-polyester (II) is obtainable by ring-opening polymerization of cyclic esters selected from the group consisting of (L,L)-dilactide, (D,D)-dilactide, (D,L)-dilactide, diglycolide or mixtures thereof, or lactones selected from the group consisting of β-(R)-butyrolactone, β-(S)-butyrolactone, β-rac-butyrolactone and ε-caprolactone or mixtures thereof.

The α,ω-dihydroxy-polyether (III) is selected from the group consisting of α,ω-dihydroxy-poly(oxytetramethylene), α,ω-dihydroxy-poly(oxyethylene) and copolymers of ethylene glycol and propylene glycol.

The α,ω-dihydroxy-polyester (IV) is obtainable by transesterification of α,ω-dihydroxy[oligo(3-(R)-hydroxybutyrate)ethylene-oligo(3-(R)-hydroxybutyrate)] (I), which is referred to hereinafter as PHB diol (IV), with diglycolide dilactide or caprolactone or mixtures thereof, the trans-esterification preferably being carried out in the presence of a catalyst. In the following reaction scheme, m is 1 to 50, n is 1 to 50, x+y is 1 to 50.

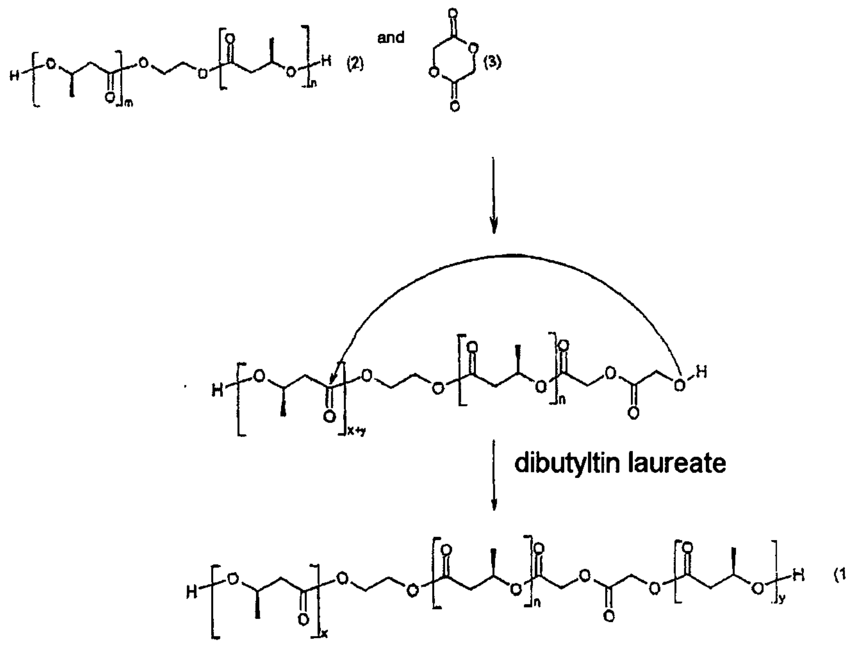

(2) and (3)

(1

Preferred catalysts are transesterification catalysts in particular based on tin, e.g. dibutyltin dilaurate. The diol preferably has a molecular weight of from 500 to 10'000 daltons. The diol (1) preferably has a total glycolide content of up to 40 mol%, particularly preferably up to 30 mol%. A preferred diol of the invention is α,ω-dihydroxy[oligo(3-R-hydroxybutyrate)-stat-glycolide)ethyleneoligo(3R)-hydroxybutyrate-stat-glycolide) or the corresponding stat-lactide or stat-caprolactate compounds if dilactide or caprolactone is used instead of diglycolide.

Further suitable α,ω-dihydroxypolyesters (II) are oligomers of α-, β-, γ- and ω-hydroxy carboxylic acids and their cooligomers which are obtained by ring-opening polymerization of cyclic esters or lactones. Preferred cyclic esters of this type are (L,L)-dilactide, (D,D)-dilactide, (D,L)-dilactide, diglycolide or the preferred lactones such as β-(R)-butyrolactone, β-(S)-butyrolactone, β-rac-butyrolactone and ε-caprolactone or mixtures thereof. The ring opening takes place with aliphatic diols such as ethylene glycol or longer-chain diols. The molecular weight of the resulting macrodiol is determined by the stoichiometrically employed amount of these diols.

The ring-opening polymerization of the cyclic esters or lactones preferably takes place without diluent in the presence of a catalyst, for example $SnO(Bu)_2$ at 100°C to 160°C. The resulting macrodiols have molecular weights of about 300-10'000 daltons. The macrodiols prepared from mixtures of cyclic esters or lactones have a microstructure which depends on the amount of catalyst and which is statistical or alternating in the distribution of the monomeric components between block form. The distributions statistics have an influence on the physical properties. Examples of such esters which are obtained by ring-opening polymerization of cyclic esters and lactones in the presence of a catalyst and which can be used to prepare the block copolymers are α,ω-dihydroxy-[poly(L-lactide)-ethylene-poly(L-lactide)]; α,ω-dihydroxy-[oligo(3-(R)-hydroxybutyrate-ran-3-(S)-hydroxybutyrate)-ethylene-oligo(3-(R)-hydroxybutyrate-ran-3-(S)-hydroxybutyrate)]; α,ω-dihydroxy-[oligo(glycolide-*ran*-ε-caprolactone)-ethylene-oligo(glycolide-*ran*-ε-caprolactone)]; α,ω-dihydroxy-[oligo(L)-lactide-*ran*-ε-caprolactone)-ethylene-oligo(L)-lactide-*ran*-ε-caprolactone)]; α,ω-dihydroxy-[oligo(L)-lactide-*ran*-glycolide)-ethylene-oligo(L)-lactide-*ran*-glycolide)]; α,ω-dihydroxy-[oligo(3-(R)-hydroxybutyrate-*ran*-3-(S)-hydroxybutyrate-*ran*-glycolide)-ethylene-oligo(3-(R)hydroxybutyrate-*ran*-3-(S)hydroxybutyrate-*ran*-glycolide); α,ω-dihydroxy-[oligo-3-(R)-hydroxybutyrate-*ran*-3-(S)-hydroxybutyrate-*ran*-L-lactide-ethylene-oligo(3-(R)-hydroxybutyrate-*ran*-(S)-hydroxybutyrate-*ran*-L-lactide)] and α,ω-hydroxy-[oligo(3-(R)-hydroxybutyrate-*ran*-3-(S)-hydroxybutyrate-*ran*-ε-caprolactone)ethylene-oligo(3-(R)-hydroxybutyrate-*ran*-3-(S)-hydroxybutyrate-*ran*-ε-caprolactone)].

The ring-opening polymerization for preparing these macrodiols can also take place without catalyst. Diisocyanates suitable for preparing the polyurethane variant of the block copolymers are in particular hexamethylene diisocyanate, 2,2,4-trimethylhexamethylene diisocyanate, cyclohexyl 1,4-diisocyanate, cyclohexyl 1,2-diisocyanate, isophorone diisocyanate, methylenedicyclohexyl diisocyanate and L-lysine diisocyanate methyl ester.

Diacid halides particularly suitable for preparing the polyester variant of the block copolymers are those of oxalic acid, malonic acid, succinic acid, glutaric acid, adipic acid, pimelic acid, suberic acid, azelaic acid, trimethyladipic acid, sebacic acid, dodecanediacid, tetradecanedioic acid and hexadecanedioic acid.

Reaction to give the polymer of the invention takes place almost quantitatively. It has moreover been found

that incorporation of the dilactide, diglycolide and/or caprolactone units results in the polymers of the invention being soluble in methylene chloride. It is thus possible to remove impurities by filtration. A cost-effective process with which the polymer of the invention can be prepared with high purity is provided thereby. The first block unit is the α,ω-dihydroxy-polyester (IV) and the second block unit is either the diol (I), the α,ω-dihydroxy-polyester (II), α,ω-dihydroxy-polyether (III) or the same α,ω-dihydroxy-polyester (IV).

A particularly preferred block copolymer is poly[poly[α,ω-dihydroxy-[oligo(3-(R)-hydroxybutyrate)-stat-glycolide)-ethylene-oligo-(3-(R)-hydroxybutyrate-stat-glycolide)]alt-2,2,4-trimethylhexamethylene 1,6-diisocyanate]-co-poly[dihydroxy[oligo-glycolide-*ran*-ε-caprolactone)-ethylene-(oligo-glycolide-*ran*-ε-caprolactone)]alt-2,2,4-trimethylhexamethylene 1,6-diisocyanate] of the formula

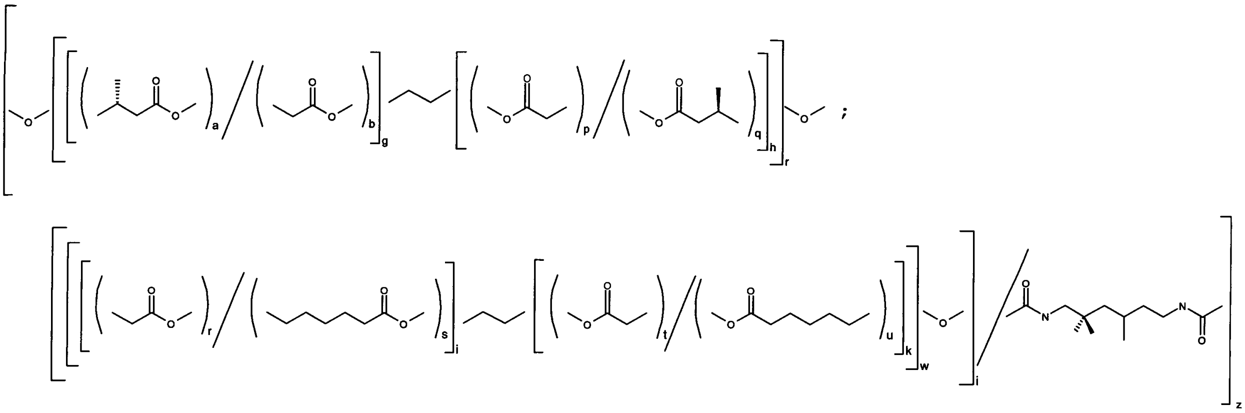

where a = 1 to 50, b = 1 to 10, g = 1 to 50, h = 1 50, i = 1 to 50, k = 1 to 50, w = 1 to 50, p = 1 to 10, q = 1 to 50, r = 1 to 10, s = 1 to 50, t = 1 to 10, u = 1 to 50 and z = 1 to 50. Further preferred polymers are identical to the abovementioned with the exception that the glycolide unit of the polymer is replaced by the corresponding lactide or caprolactone.

The membranes comprising glycolide units which are particularly preferred are those degradable in five to six days within the human or animal body. Further preferred membranes are those whose degradation takes place over months or years. The rate of degradation depends primarily on the number of diglycolide or glycolide units. On storage in a neutral buffer solution at 37°C, the molecular weight decreases with time as a function of the glycolide content. The use of dilactide or caprolactone units does not change the rate of degradability of the polymers of the invention in the body.

The membranes can comprise more than one layer. In a preferred embodiment the membrane comprises only single layer.

It has been found that the membrane according to the present invention has an exceptionally good biocompatibility. In addition, it is possible through the incorporation of the glycolide or diglycolide units to control the hydrolytic and biological rate of degradability of the membrane. The degradability of the block copolymer outside the body can be increased, besides the incorporation of glycolide or diglycolide units, by (L,L)-dilactide, (D,D)-dilactide, (D,L)-dilactide or mixtures thereof.

In a preferred embodiment the membranes have a service time. This service time may be defined as the time which elapses from the time point in which the membrane comes to the first time into contact with water and the point in time, when the mechanical properties of the material begins to drop down, and the properties of the membrane become insufficient to fulfill its task. This is, when the membrane begins to loose mass, is getting brittle or the pore size and shape alters. These changes go in parallel with the changes of the parameter in table 1 above. The service time of the said membranes may be 5 days up to 2 years. Preferably, the service life time of a membrane according to the present invention is between 14 to 28 days. This range is suitable for tissue engineering. On a molecular basis this is the time point, when the macromolecules, building up our material drop below an average molecular weight of about 10 000 Da.

Membranes of the present invention can be employed as surgical aids or can be comprised in surgical aids. A major advantage of the use of membranes as surgical aids or implants is their controllable degradability. Because this degradability can be tailored according to the needs of the particular situation a second surgery, in order to remove an implant that is no longer needed, can be avoided. Such second surgeries are a prominent source of complications, e.g. causing unwanted inflammation, infections etc., besides the fact that they drive up costs.

Since the membranes can be prepared in various forms, e.g. flat, tubular or as capsules, the membranes provide an excellent flexibility in terms of possible uses. For instance, the implants may be in the form of a tube. The tube may be rigid or flexible. The tubes may have circular, elliptical and polygonal cross sections.

The implant material may have a porous structure for particular uses. It is possible with the implants of the invention to regenerate a functional vessel wall or a nerve. It is possible by a coating with functional vessel cells (endothelial

cells) to avoid a thrombotic occlusion on long-term use, i.e. the biocompatible polymer can be a replacement. The implant may also have a capsule shape to receive pharmaceutical active substances or diagnostics also in the form of particles.

In a preferred embodiment the membrane of the present invention comprises at least one pharmaceutically active compound or diagnostic aid. Such compounds include hormones, enzymes, cytokines, growth factors, antiinflammatory drugs, e.g. steroids or non steroidal antiinflammatory drugs (NSAIDs) and the like. These compounds can be entrapped within the membrane or they can be covalently bound to the membrane. Preferably, they are covalently bound to the membrane.

In addition, further possible uses in appropriate physical and or biological form are in medical dental, micro- or nanotechnologies.

The present invention also relates to a method according to claim 15 for preparing a membrane of the present invention. Preferred embodiments are the subject-matter of dependent claims 16 to 20.

A method for preparing a membrane according to the present invention comprises the following steps, first a biocompatible block copolymer is dissolved in a suitable solvent, e.g. dioxane, second the block copolymer solution is applied on a carrier. If the membrane to be prepared is flat, a glass plate can be used as a carrier. Third, after the block copolymer solution has been applied on the carrier, said carrier is immersed in a non-solvent which is miscible with the solvent, resulting in the porous membrane, the pores of which having size in the range of 0.2 to 20.0 μm.

The carrier which is employed depends on the desired shape of the membrane. Apart from glass plates for flat membranes, tubular forms can be used to prepare membranes of cylindrical shape.

Instead of immersing the carrier with the applied block copolymer solution in the non-solvent, the latter can also be sprayed on said carrier.

Suitable solvents for the block copolymers are dioxane, chloroform, dimethylcarbonate and butanon. A preferred solvent is 1,4-dioxane. It can easily be removed from the polymer and has the least toxic side effects.

As non-solvents may be used water, methanol and ethanol.

A preferred non-solvent is water. A preferred solvent/non-solvent pair is 1,4-dioxane and water.

According to claims 21 and 22 the present invention also relates to sheet-like structures comprising a membrane.

Said sheet-like structure may comprise a membrane and for instance a mesh. The fibers forming the mesh further enhance the mechanical stability of the structure.

In the following the figures are briefly described:

Figure 1A illustrates the step of sucking the polymer solution into a tube preparing a membrane having a tubular form using the "negative method".

Figure 1B shows a further step of the "negative method" where the tube with the remaining polymer solution is horizontally mounted and rotated in order to achieve an even layer of the polymer.

Figure 2 illustrates the porous structure of a membrane prepared from 16% polymer solution (solvent dioxan).

Figure 3 shows the porous structure of a membrane prepared from 14% polymer solution (solvent dioxan).

Figure 4 shows illustrates the porosity of a membrane prepared from 14% polymer solution (solvent dioxan)

Figure 5 shows a membrane prepared from 14% polymer solution (solvent dioxan).

Figure 6 shows a cross section of a membrane.

Figure 7 shows a cross section of a membrane at a higher magnification.

Figure 8 illustrates a sheet-like structure comprising a membrane and a mesh.

Figure 9 again shows a sheet-like structure.

Figure 10 illustrates the combination of a membrane and a mesh forming a sheet-like structure.

Figure 11 shows a sheet-like structure comprising a membrane and a mesh at a higher magnification.

Figure 12 shows a sheet-like structure at a high magnification.

Figure 13 shows a welding seam of two membranes forming a bag-like structure.

Figure 14 shows a section of two membranes which have been welded.

Figure 15 shows a welding seam of a membrane capsule.

Figure 16 shows fibroblast 3T3 cells after 72h in the eluate (a) control (b) membrane.

Figure 17 shows a fluorescein diacetate staining of live cells on membrane, 72h.

Figure 18 shows a fluorescein diacetate staining of live cells on membrane, 12d.

**Example 1**

*Manufacturing a membrane*

To obtain a membrane, the molecular weight (Mw) of the biocompatible block-copolymer must exceed a lower limit 50’000 Da < Mw. Otherwise the result will be a transparent film and not a membrane. The polymer is dissolved in dioxane at a concentration of 6-25% wt/wt. Four coagulation baths are used successively. The first one with EtOH (or an alternative non-solvent), the second with MeOH (or an alternative non-solvent), the third one with distilled water (in which few detergent is added) and the last one with distilled water. On a clean glass plate, with help of a doctor’s blade allowing to doctor a film of 500pm is used. It is pushed gently over the glass’ surface. The glass plate with the layer of polymer solution on it is then contacted with the non solvent in the first bath, where the transparent film becomes opaque resulting in a porous membrane. The film is then successively washed in the remaining bath’s and at the end of the fourth bath, the membrane is dried on air or in the vacuum oven. The resulting pores have a size in the range of 0.2$\mu$m to 20$\mu$m. Alternatively the film which is doctored on the glass plate may first be contacted with non solvent by spraying the non solvent as a fine haze over the surface. This process can be transferred to an enlarged process of membrane production on the surface of a rotating cylinder.

*Production of a membrane tube*

To receive a perfect membrane tube, the molecular weight of the applied polymer should exceed 70’000 dalton. The polymer is solved in dioxane at a concentration of 25%.

Three baths are necessary: the first one with MeOH cooled down to -20°C (+/- 2°C). The temperature should be as constant as possible. The second one with EtOH held at 0°C and the last one filled with distilled water at room temperature.

There are two methods to produce tubes:

The first one is the so called negative method.

A glass tube with the right internal diameter is provided as well as an internal tube (PTFE or glass is needed to prevent the resulting membrane from sticking together)

The beforehand prepared solution is sucked into the tube (Figure 1A). The spare solvent drips out back into the container. The resuming solution adheres on the tube’s wall.

The glass tube is now mounted horizontally into a stirring motor and turned at 2000 turns per minute for several seconds (Figure 1B).

Consequently, the resulting layer on the glass tube’s wall is of a homogeneous thickness.

Afterwards, the tube is dropped instantly into the cooled first bath and remains there for approximately five minutes. After that, the tube is transferred for another five minutes into the second bath and finally into the last one (before putting it into the last one, the internal tube has to be adjusted).

The second method is called the positive method.

Here, we directly use a bar with the right diameter:

The prepared polymer solution should be as viscous as possible since it has to adhere on the pole’s surface. The bar is dipped into the polymer solution and immediately put into the first bath where the thin layer of the dioxane solution freezes instantly. After 30 minutes, it is then transferred into the second bath and finally put into the third where the membrane either tears or dismantles from the bar.

*Porosity and surface structure*

The porosity can be adjusted by altering the solution's concentration. Starting with a solution of 10% and ending with one with over 25% polymer solved in dioxane.
It is shown that an increasing concentration comes along with a reduction of the pore size.

*Combination of membrane and electrospun fibres*

The resulting membranes are homogeneously porous but not very strong. Therefore, a combination of membrane and a strong electrospun fibre network was aspired. (With the same polymer). Typical examples of such sheet-like structures comprising a membrane and a mesh of fibres are shown in Figures 8 to 12.

*Membrane bags*

Several applications demand for a closed structure akin to a bag wherein cells or other material can be stored.
Bags can be obtained by fusing two congruent membranes together. A heated bar (approx 150°C) was used to melt the edges together. In order to prevent the membrane from melting, it is moistened with distilled water.

**Example 2**

Mass transfer coefficients D were determined for a membrane at room temperature and water, with a series of fluorescent polysaccharides (FITC-Ficoll) with different molecular weights The following values were found for a 65pm thick membrane:

| Tracer | MW | N | Mass Transfer coefficient D in $mm^2$/sec |
|---|---|---|---|
| Iohexol | 821 | 6 | 8.3 ± 1.6 x 10-4 |
| FITC-Ficoll 16 | 5000 | 2 | 5.3 ± 1.6 x 10-4 |
| FITC-Ficoll 30 | 22000 | 2 | 3.2 ± 0.5 x 10-4 |
| FITC-Ficoll 50 | 72000 | 2 | 2.4 ± 0.1 x 10-4 |
| FITC-Ficoll 120 | 561000 | 2 | 2.0 ± 0.5 x 10-4 |

**Example 3**

Fluorescence microscope analysis of cell seeded polymers:

A live-death stain was performed on the polymer variants. Fluorescein diacetate (FDA) and ethidium bromide (EB) were used to indicate live and dead cells respectively. At time points of 2d, 4d and 12d, two wells for each variant and control were examined. The medium was removed. Cell seeding of polymer variants and the cells were washed twice with 200μl sterile phosphate buffered saline (PBS). 2ml 70% ethanol was added to the control cells and incubated for 5 minutes to act as a negative control.

After this time the ethanol was removed. The cells were then stained with Fluorescein-diacetate (FDA) (2.5μl/ml) and ethidium bromide (EB) (10μl/ml) in PBS for 1 minute at room temperature. Staining was removed and washed twice with sterile PBS. Live and dead cells were analysed using fluorescence microscopy.
**Cytotoxicity test:** Images of the cells after 72h are detailed in figure 16. All variants exhibit good response to the eluate compared to the control indicating that any released by-products after 24h are not very toxic to the cells. Continued good cell growth was observed at further time points (not shown).

**Claims**

1. Membrane comprising a biocompatible block copolymer having at least two block units obtainable by linear polycondensation in the presence of diisocyanate, diacid halide or phosgene
of first block unit being an α,ω-dihydroxy-polyester (IV) with a second block unit selected from the group consisting of a diol (I), an α,ω-dihydroxy-polyester (II), an α,ω-dihydroxy-polyether (III), and the same α,ω-dihydroxy-polyester (IV),
wherein the diol (I) is obtainable by transesterification of poly-[(R)-(3)-hydroxybutyric acid] or copolymers thereof

with 3-hydroxyvaleric acid with ethylene glycol,
wherein the α,ω-dihydroxy-polyester (II) is obtainable by ring-opening polymerization of cyclic esters selected from the group consisting of (L,L)-dilactide, (D,D)-dilactide, (D,L)-dilactide, diglycolide or mixtures thereof, or lactones selected from the group consisting of β-(R)-butyrolactone, β-(S)-butyrolactone, β-rac-butyrolactone and ε-caprolactone or mixtures thereof,
wherein the α,ω-dihydroxy-polyether (III) is selected from the group consisting of α,ω-dihydroxy-poly(oxytetramethylene), α,ω-dihydroxy-poly(oxyethylene) and copolymers of ethylene glycol and propylene glycol,
wherein the α,ω-dihydroxy-polyester (IV) is obtainable by trans-esterification of α,ω-dihydroxy [oligo (3-(R)-hydroxybutyrate)ethylene-oligo (3-(R)-hydroxybutyrate)] with diglycolide and/or dilactide and/or caprolactone or mixtures thereof, and
**characterized in that** the membrane comprises regularly distributed pores, said pores having a size in the range of 0.2 to 20.0 μm.

**2.** Membrane according to claim 1, wherein the first block unit is the a,ω-dihydroxy-polyester (IV) and the second block unit is the diol (I).

**3.** Membrane according to claim 1, wherein the first block unit is the α,ω-dihydroxy-polyester (IV) and the second block unit is the α,ω-dihydroxy-polyester (II).

**4.** Membrane according to claim 1, wherein the first block unit is the α,ω-dihydroxy-polyester (IV) and the second block unit is the same α,ω-dihydroxy-polyether (III) .

**5.** Membrane according to claim 1, wherein the first block unit is the α,ω-dihydroxy-polyester (IV) and the second block unit is the α,ω-dihydroxy-polyester (IV).

**6.** Membrane according to claim 1 to 5, wherein the pores have a size in the range of 0.2 to 10.0μm.

**7.** Membrane according to claim 1 to 6, wherein said membrane is permeable or semipermeable.

**8.** Membrane according to claim 1 to 7, wherein the block copolymer is poly[poly[α,ω-dihydroxy-[oligo(3-(R)-hydroxybutyrate)-stat-glycolide)-ethylene-oligo-(3-(R)-hydroxybutyrate-stat-glycolide)]alt-2,2,4-trimethylhexamethylene-1,6-diisocyanate]-co-poly-[dihydroxy[oligo-glycolide-ran-ε-caprolactone)-ethylene-(oligo-glycolide-ran-e-caprolactone)]alt-2,2,4-trimethylhexamethylene-1,6-diisocyanate].

**9.** Membrane according to claim 1 to 7, wherein the block copolymer is poly[poly[α,ω-Dihydroxy-[oligo(3-(R)-hydroxybutyrat)-co-ε-caprolacton)-ethylen-oligo-(3-(R)-hydroxybutyrat-co-ε-caprolacton)]alt-2,2,4-trimethylhexamethylene-1,6-diisocyanat]-copoly[dihydroxy[oligo-glykolid-ran-ε-caprolacton)-ethylen-(oligo-glykolid-ran-ε-caprolacton)]alt-2,2,4-trimethylhexamethylene-1,6-diisocyanat].

**10.** Membrane according to claim 1 to 9, wherein said membrane comprises a single layer.

**11.** Membrane according to claim 1 to 10, wherein said membrane is hydrolytically degradable and has a controllable service life time and keeps its performance from 5 days to 2 years, preferably between 14 and 28 days.

**12.** Membrane according to claim 1 to 11, wherein said membrane has a tubular form.

**13.** Membrane according to claims 1 to 12, wherein said membrane comprises at least one pharmaceutically active compound or diagnostic aid.

**14.** A surgical aid intended to be fixed in and on the human or animal body, comprising said membrane as claimed in claims 1 to 13.

**15.** Method for preparing a membrane as claimed in claim 1, wherein

a) a biocompatible block copolymer is dissolved in a solvent; and
b) said block copolymer solution is applied on a suitable, shaped carrier; and
c) said carrier is immersed in a non-solvent which is miscible with the solvent,

resulting in the membrane comprising pores having a size in the range of 0.2 - 20.0 μm.

**16.** Method according to claim 15, wherein the non-solvent is sprayed on said carrier.

**17.** Method according to claim 15 or 16, wherein the solvent is selected from the group consisting of dioxane, chloroform, dimethylcarbonate and butanon.

**18.** Method according to claim 15, wherein the solvent is 1,4-dioxane.

**19.** Method according to claim 15 or 16, wherein the non-solvent is selected from the group consisting of water, methanol and ethanol.

**20.** Method according to claim 15, wherein the non-solvent is water.

**21.** Sheet-like structure, wherein said structure comprises a membrane according to claim 1 to 14.

**22.** Sheet-like structure, comprising a membrane according to claim 1 to 14 and a mesh.

**Patentansprüche**

**1.** Membran, die ein biokompatibles Block-Copolymer umfasst, das mindestens zwei Blockeinheiten hat, die durch lineare Polykondensation, in Gegenwart von Diisocyanat, Diacidhalid oder Phosgen, einer ersten Blockeinheit gewonnen werden kann, die ein α,ω-Dihydroxy-polyester (IV) ist, mit einer zweiten Blockeinheit, wobei die zweite Blockeinheit ausgewählt wird aus der Gruppe, die aus einem Diol (I), einem α,ω-Dihydroxy-polyester (II), einem α,ω-Dihydroxy-polyether (III) und demselben α,ω-Dihydroxy-polyester (IV) besteht,
wobei das Diol (I) durch Transesterifikation von Poly-[(R)-(3)-hydroxybuttersäure] oder Copolymeren derselben mit 3-Hydroxyvaleriansäure mit Ethylenglycol gewonnen werden kann,
wobei das α,ω-Dihydroxy-polyester (II) durch ringöffnende Polymerisation von zyklischen Estern gewonnen werden kann, die aus der Gruppe ausgewählt werden, die aus (L,L)-Dilactid, (D,D)-Dilactid, (D,L)-Dilactid, Diglykolid oder Mischungen derselben besteht, oder aus Lactonen, die aus der Gruppe ausgewählt werden, die aus β-(R)-Butyrolacton, β-(S)-Butyrolacton, β-rac-Butyrolacton und ε-Caprolacton oder Mischungen derselben besteht,
wobei der α,ω-Dihydroxy-polyether (III) aus der Gruppe ausgewählt wird, die aus α,ω-Dihydroxy-poly(oxytetramethylen), α,ω-Dihydroxy-poly(oxyethylen) und Copolymeren von Ethylenglykol und Propylenglykol besteht,
wobei der α,ω-Dihydroxy-polyester (IV) durch Transesterifikation von α,ω-Dihydroxy-[oligo (3-(R)-hydroxy-butyrat)ethylen-oligo(3-(R)-hydroxy-butyrat)] mit Diglykolid und/oder Dilactid und/oder Caprolacton oder Mischungen derselben gewonnen werden kann, und
**dadurch gekennzeichnet, dass** die Membran regelmäßig verteilte Poren umfasst, wobei die Poren eine Größe im Bereich von 0,2 to 20,0 μm haben.

**2.** Membran nach Anspruch 1, wobei die erste Blockeinheit der α,ω-Dihydroxy-polyester (IV) ist und die zweite Blockeinheit das Diol (I) ist.

**3.** Membran nach Anspruch 1, wobei die erste Blockeinheit der α,ω-Dihydroxy-polyester (IV) ist und die zweite Blockeinheit das α,ω-Dihydroxy-polyester (II) ist.

**4.** Membran nach Anspruch 1, wobei die erste Blockeinheit der α,ω-Dihydroxy-polyester (IV) ist und die zweite Blockeinheit derselbe α,ω-Dihydroxy-polyether (III) ist.

**5.** Membran nach Anspruch 1, wobei die erste Blockeinheit der α,ω-Dihydroxy-polyester (IV) ist und die zweite Blockeinheit der α,ω-Dihydroxy-polyester (IV) ist.

**6.** Membran nach Anspruch 1 bis 5, wobei die Poren eine Größe im Bereich von 0,2 bis 10,0 μm haben.

**7.** Membran nach Anspruch 1 bis 6, wobei die Membran permeabel oder semipermeabel ist.

**8.** Membran nach Anspruch 1 bis 7, wobei das Block-Copolymer Poly[poly[α,ω-dihydroxy-[oligo(3-(R)-hydroxybutyrat)-stat-glykolid)-ethylen-oligo-(3-(R)-hydroxybutyrat-stat-glykolid)]alt-2,2,4-trimethylhexamethylen-1,6-diisocyanat]-co-poly-[dihydroxy[oligo-glykolid-ran-ε-caprolacton)-ethylen-(oligo-glykolid-ran-ε-caprolacton)]alt-2,2,4-trimethylhexamethylen-1,6-diisocyanat] ist.

**9.** Membran nach Anspruch 1 bis 7, wobei das Block-Copolymer Poly[poly[α,ω-dihydroxy-[oligo(3-(R)-hydroxybutyrat)-co-ε-caprolacton)-ethylen-oligo-(3-(R)-hydroxybutyrat-co-ε-caprolacton)]alt-2,2,4-trimethylhexamethylen-1,6-diisocyanat]-copoly-[dihydroxy[oligo-glykolid-ran-ε-caprolacton)-ethylen-(oligo-glykolid-ran-ε-caprolacton)]alt-2,2,4-trimethylhexamethylen-1,6-diisocyanat] ist.

**10.** Membran nach Anspruch 1 bis 9, wobei die Membran eine einzige Schicht umfasst.

**11.** Membran nach Anspruch 1 bis 10, wobei die Membran hydrolytisch abbaubar ist und eine steuerbare Gebrauchsdauer hat und seine Leistungsfähigkeit von 5 Tagen bis 2 Jahren behält, vorzugsweise zwischen 14 und 28 Tagen.

**12.** Membran nach Anspruch 1 bis 11, wobei die Membran eine Rohrform besitzt.

**13.** Membran nach Anspruch 1 bis 12, wobei die Membran mindestens eine pharmazeutisch aktive Verbindung oder diagnostisches Hilfsmittel umfasst.

**14.** Chirurgisches Hilfsmittel, das dafür vorgesehen ist, in oder auf dem menschlichen oder tierischen Körper befestigt zu werden, das die Membran nach einem der Ansprüche 1 bis 13 enthält.

**15.** Verfahren zum Herstellen einer Membran nach Anspruch 1, wobei

a) ein biokompatibles Block-Copolymer in einem Lösungsmittel aufgelöst ist; und
b) die Block-Copolymer-Lösung auf einen geeigneten, geformten Träger aufgetragen wird; und
c) der Träger in ein Nichtlösungsmittel eingetaucht wird, das mit dem Lösungsmittel mischbar ist,

was dazu führt, dass die Membran Poren enthält, die eine Größe im Bereich von 0,2 - 20,0 μm haben.

**16.** Verfahren nach Anspruch 15, wobei das Nichtlösungsmittel auf den Träger aufgesprüht wird.

**17.** Verfahren nach Anspruch 15 oder 16, wobei das Lösungsmittel aus der Gruppe bestehend aus Dioxan, Chloroform, Dimethylcarbonat und Butanon ausgewählt wird.

**18.** Verfahren nach Anspruch 15, wobei das Lösungsmittel 1,4-Dioxan ist.

**19.** Verfahren nach Anspruch 15 oder 16, wobei das Nichtlösungsmittel aus der Gruppe bestehend aus Wasser, Methanol und Ethanol ausgewählt wird.

**20.** Verfahren nach Anspruch 15, wobei das Nichtlösungsmittel Wasser ist.

**21.** Blattförmige Struktur, wobei die Struktur eine Membran nach Anspruch 1 bis 14 umfasst.

**22.** Blattförmige Struktur, die eine Membran nach Anspruch 1 bis 14 und ein Gitter umfasst.

## Revendications

**1.** Membrane comprenant un copolymère en bloc biocompatible ayant au moins deux unités en bloc que l'on peut obtenir par une polycondensation linéaire, en présence d'un diisocyanate, d'un halogénure de diacide ou d'un phosgène,
d'une première unité en bloc, laquelle est un α,ω-dihydroxy-polyester (IV), avec une deuxième unité en bloc choisie dans le groupe consistant en un diol (I), un α,ω-dihydroxy-polyester (II), α,ω-dihydroxy-polyéther (III) et le même α,ω-dihydroxy-polyester (IV),
dans laquelle le diol (I) peut être obtenu par une trans-estérification de poly-[(R)-(3)-acide hydroxybutyrique] ou de copolymères de celui-ci avec de l'acide 3-hydroxyvalérique avec de l'éthylène glycol,
dans laquelle le α,ω-dihydroxy-polyester (II) peut être obtenu par une polymérisation à ouverture d'anneau d'esters cycliques, choisis dans le groupe consistant en le (L,L)-dilactide, le (D,D)-dilactide, le (D,L)-dilactide, un diglycolide

ou des mélanges de ceux-ci, ou bien de lactones choisies dans le groupe consistant en la $\beta$-(R)-butyrolactone, la $\beta$-(S)-butyrolactone, la $\beta$-rac-butyrolactone et la $\varepsilon$-caprolactone ou des mélanges de ceux-ci,
dans laquelle le $\alpha,\omega$-dihydroxy-polyéther (III) est choisi dans le groupe consistant en le $\alpha,\omega$-dihydroxy-poly(oxytétraméthylène), le $\alpha,\omega$-dihydroxy-poly(oxyéthylène) et des copolymères d'éthylène glycol et de propylène glycol,
dans laquelle le $\alpha,\omega$-dihydroxy-polyester (IV) peut être obtenu par une trans-estérification de $\alpha,\omega$-dihydroxy-[oligo(3-(R)-hydroxybutyrate)éthylène-oligo(3-(R)-hydroxy-butyrate)] avec un diglycolide et/ou un dilactide et/ou une caprolactone ou des mélanges de ceux-ci, et **caractérisée en ce que** la membrane comprend des pores distribués régulièrement, lesdits pores ayant une taille comprise dans la plage 0,2 à 20,0 $\mu$m.

**2.** Membrane selon la revendication 1, dans laquelle la première unité en bloc est le $\alpha,\omega$-dihydroxy-polyester (IV) et la deuxième unité en bloc est le diol (I).

**3.** Membrane selon la revendication 1, dans laquelle la première unité en bloc est le $\alpha,\omega$-dihydroxy-polyester (IV) et la deuxième unité en bloc est le $\alpha,\omega$-dihydroxy-polyester (II).

**4.** Membrane selon la revendication 1, dans laquelle la première unité en bloc est le $\alpha,\omega$-dihydroxy-polyester (IV) et la deuxième unité en bloc est le même $\alpha,\omega$-dihydroxy-polyéther (III).

**5.** Membrane selon la revendication 1, dans laquelle la première unité en bloc est le $\alpha,\omega$-dihydroxy-polyester (IV) et la deuxième unité en bloc est le $\alpha,\omega$-dihydroxy-polyester (IV).

**6.** Membrane selon les revendications 1 à 5, dans laquelle les pores ont une taille comprise dans la plage 0,2 à 10,0 $\mu$m.

**7.** Membrane selon les revendications 1 à 6, dans laquelle ladite membrane est perméable ou semi-perméable.

**8.** Membrane selon les revendications 1 à 7, dans laquelle le copolymère en bloc est le poly[poly[$\alpha,\omega$-dihydroxy-[oligo(3-(R)- hydroxybutyrate)-stat-glycolide)-éthylène-oligo-(3- (R)-hydroxybutyrate-stat-glycolide)]alt-2,2,4- triméthylhexaméthylène-1,6-diisocyanate]-co-poly- [dihydroxy[oligo-glycolide-ran-$\varepsilon$-caprolactone)- éthylène-(oligo-glycolide-ran-$\varepsilon$-caprolactone)]alt-2,2, 4-triméthylhexaméthylène-1,6-diisocyanate].

**9.** Membrane selon les revendications 1 à 7, dans laquelle le copolymère en bloc est le poly[poly[$\alpha,\omega$-Dihydroxy-[oligo(3-(R)-hydroxybutyrate)-co-$\varepsilon$-caprolactone)-éthylène-oligo-(3-(R)-hydroxybutyrate-co-$\varepsilon$-caprolactone)]alt-2,2,4-triméthylhexaméthylène-1,6-diisocyanate]-copoly[dihydroxy[oligo-glycolide-ran-$\varepsilon$-caprolactone)-éthylène-(oligo-glycolide-ran-$\varepsilon$-caprolactone)]alt-2, 2,4-triméthylhexaméthylène-1,6-diisocyanate].

**10.** Membrane selon les revendications 1 à 9, dans laquelle ladite membrane comprend une seule couche.

**11.** Membrane selon les revendications 1 à 10, dans laquelle ladite membrane est dégradable par hydrolyse et a une durée de vie utile contrôlable et maintient ses performances de 5 jours jusqu'à 2 ans, de préférence entre 14 et 28 jours.

**12.** Membrane selon les revendications 1 à 11, dans laquelle ladite membrane a une forme tubulaire.

**13.** Membrane selon les revendications 1 à 12, dans laquelle ladite membrane comprend au moins un composé actif, d'un point de vue pharmaceutique, ou un auxiliaire diagnostique.

**14.** Auxiliaire chirurgical destiné à être fixé dans et sur le corps humain ou animal, comprenant ladite membrane telle que revendiquée dans les revendications 1 à 13.

**15.** Procédé de préparation d'une membrane, telle que revendiquée dans la revendication 1, dans lequel

a) un copolymère en bloc biocompatible est dissout dans un solvant ; et
b) ladite solution de copolymère en bloc est appliquée sur un support approprié et façonné ; et
c) ledit support est immergé dans un non solvant qui est miscible dans le solvant,

conduisant à la membrane qui comprend des pores ayant une taille comprise dans la plage 0,2 à 20,0 $\mu$m.

**16.** Procédé selon la revendication 15, dans lequel le non solvant est pulvérisé sur ledit support.

**17.** Procédé selon les revendications 15 ou 16, dans lequel le solvant est choisi dans le groupe consistant en le dioxane, le chloroforme, le diméthyl-carbonate et la butanone.

**18.** Procédé selon la revendication 15, dans lequel le solvant est le 1,4-dioxane.

**19.** Procédé selon les revendications 15 ou 16, dans lequel le non solvant est choisi dans le groupe consistant en l'eau, le méthanol et l'éthanol.

**20.** Procédé selon la revendication 15, dans lequel le non solvant est l'eau.

**21.** Structure du type feuillet, dans laquelle ladite structure comprend une membrane selon les revendications 1 à 14.

**22.** Structure du type feuillet comprenant une membrane, selon les revendications 1 à 14, ainsi qu'une maille.

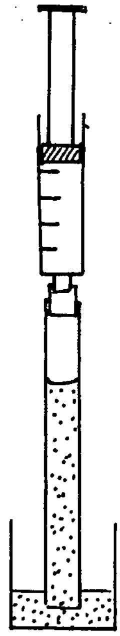

Figure 1A

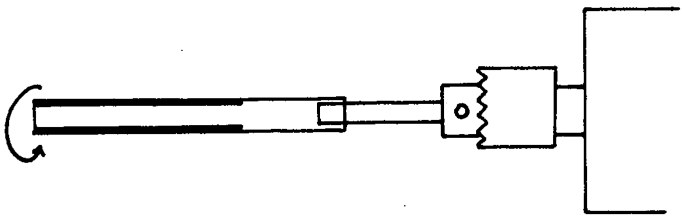

Figure 1B

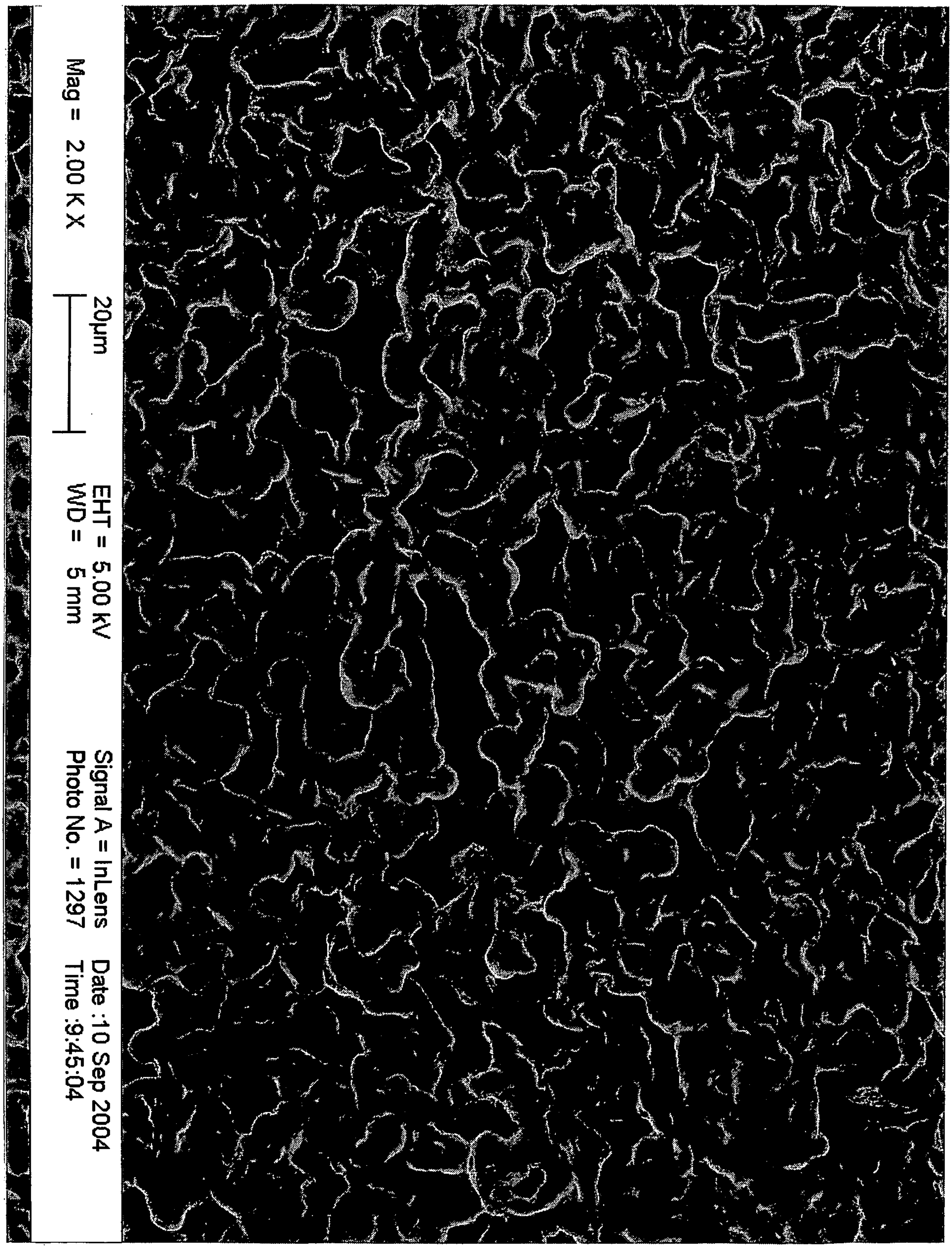
Mag = 2.00 K X
20µm
EHT = 5.00 kV
WD = 5 mm
Signal A = InLens
Photo No. = 1297
Date :10 Sep 2004
Time :9:45:04

Figure 2

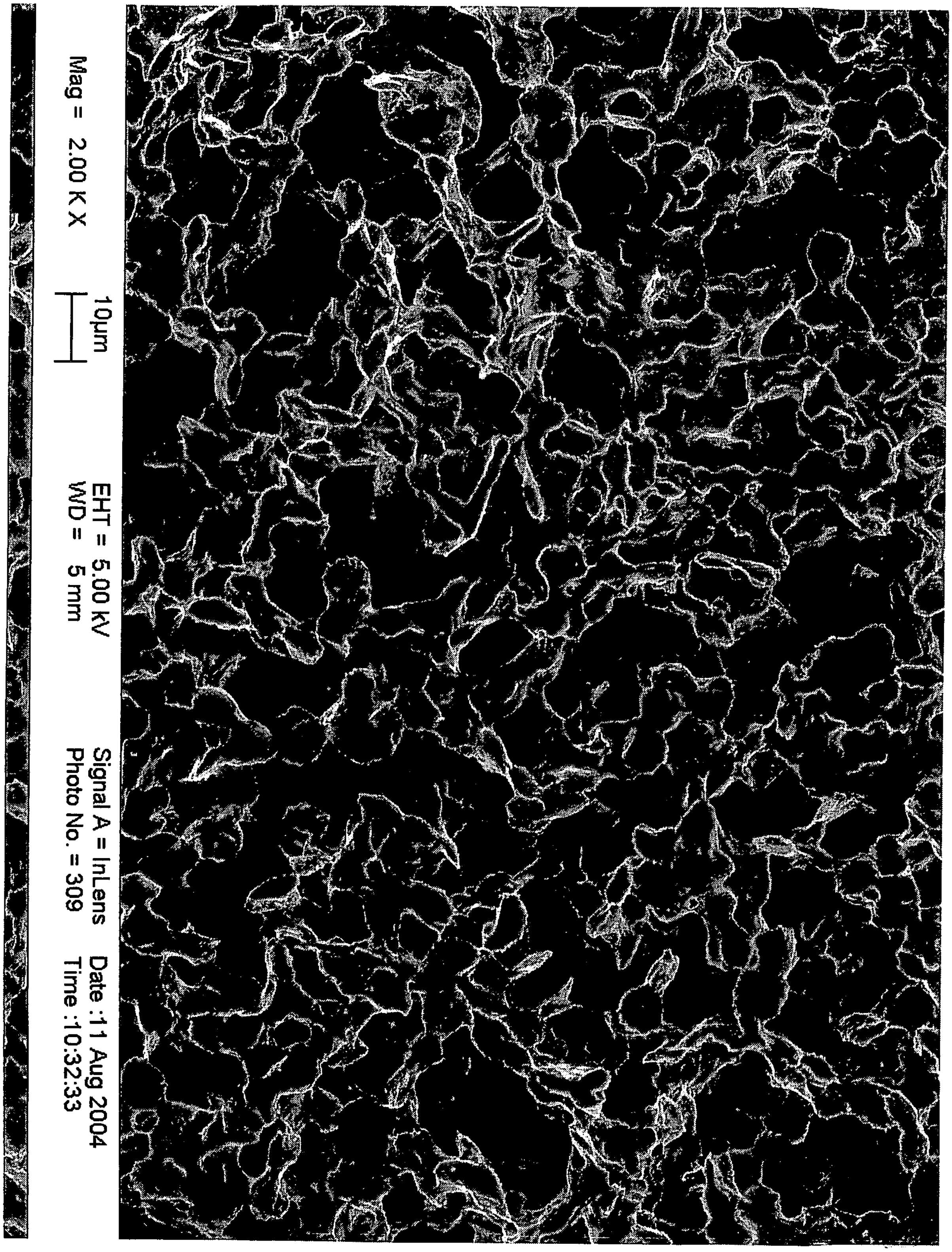
Mag = 2.00 K X
10µm
EHT = 5.00 kV
WD = 5 mm
Signal A = InLens
Photo No. = 309
Date :11 Aug 2004
Time :10:32:33

Figure 3

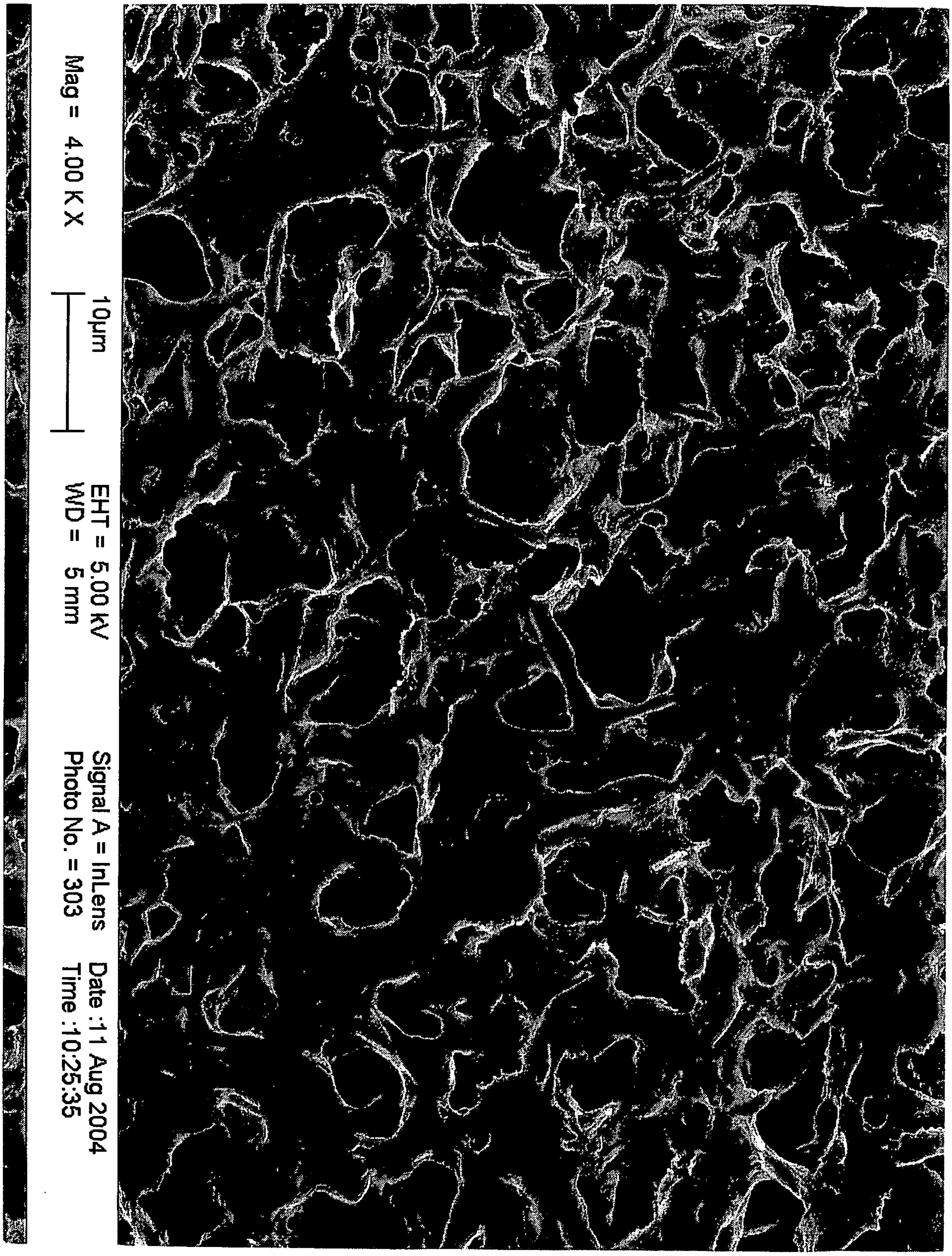
Mag = 4.00 K X
10µm
EHT = 5.00 kV
WD = 5 mm
Signal A = InLens
Photo No. = 303
Date :11 Aug 2004
Time :10:25:35

Figure 4

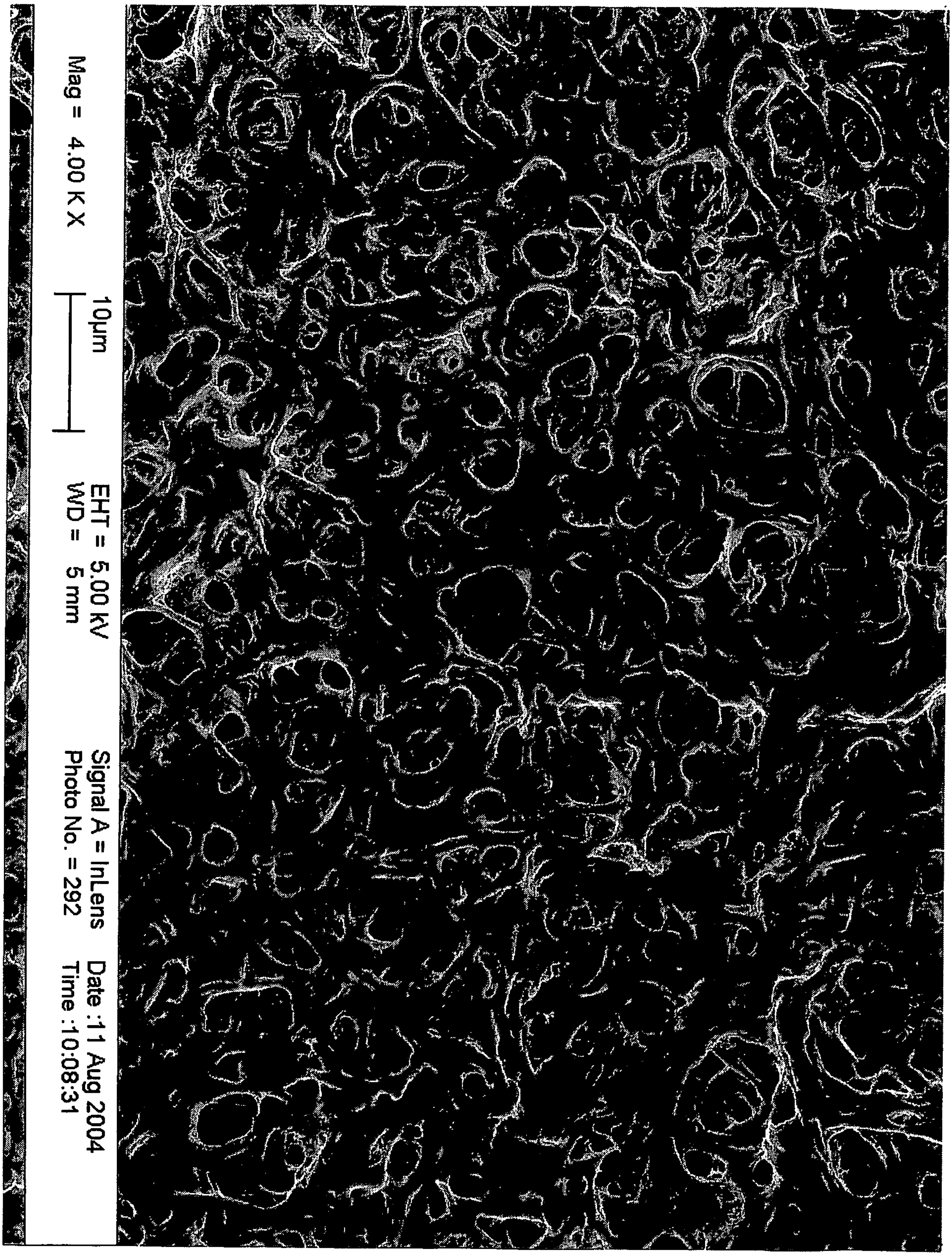
Mag = 4.00 K X
10µm
EHT = 5.00 kV
WD = 5 mm
Signal A = InLens
Photo No. = 292
Date :11 Aug 2004
Time :10:08:31

Figure 5

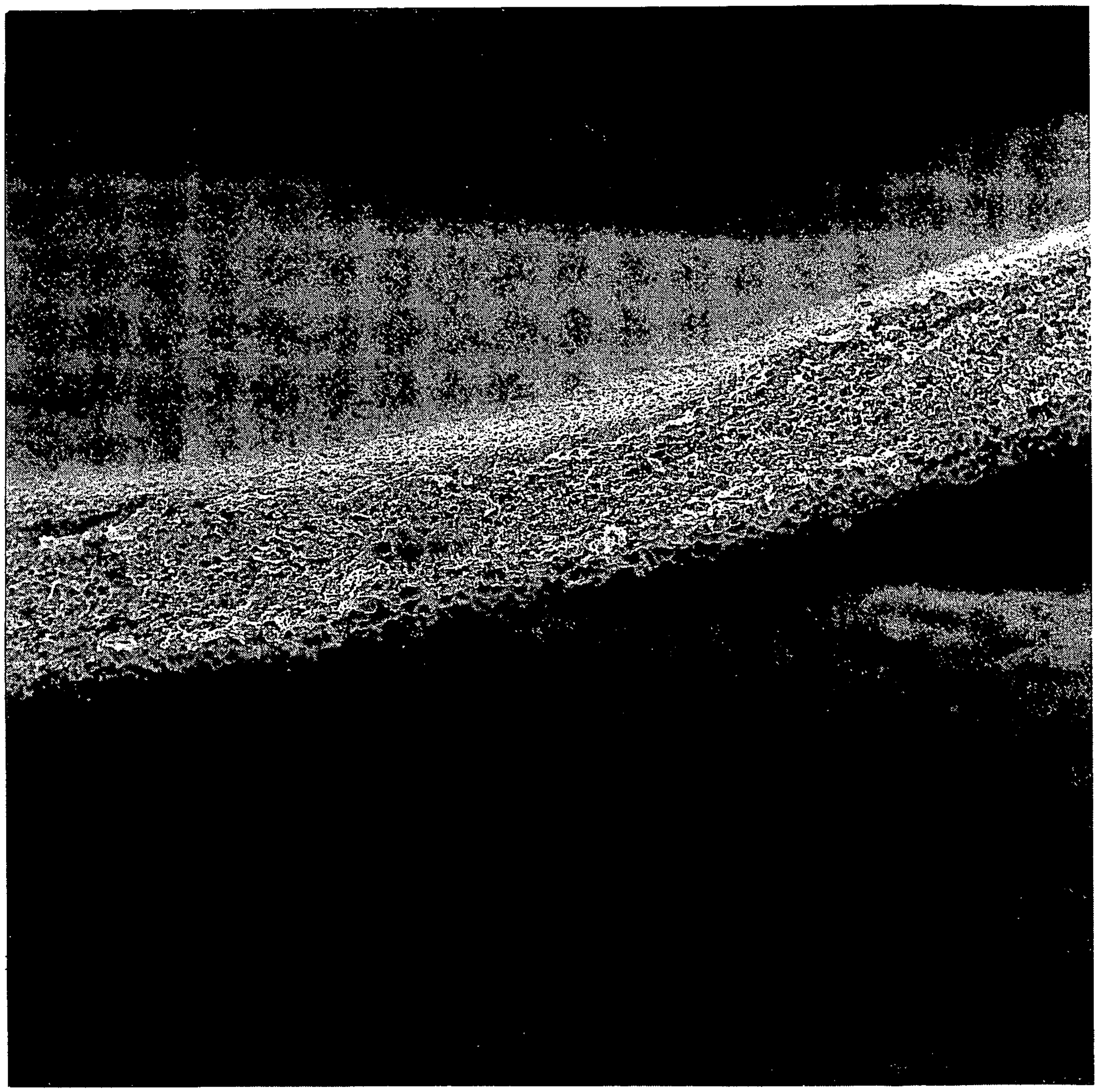

Figure 6

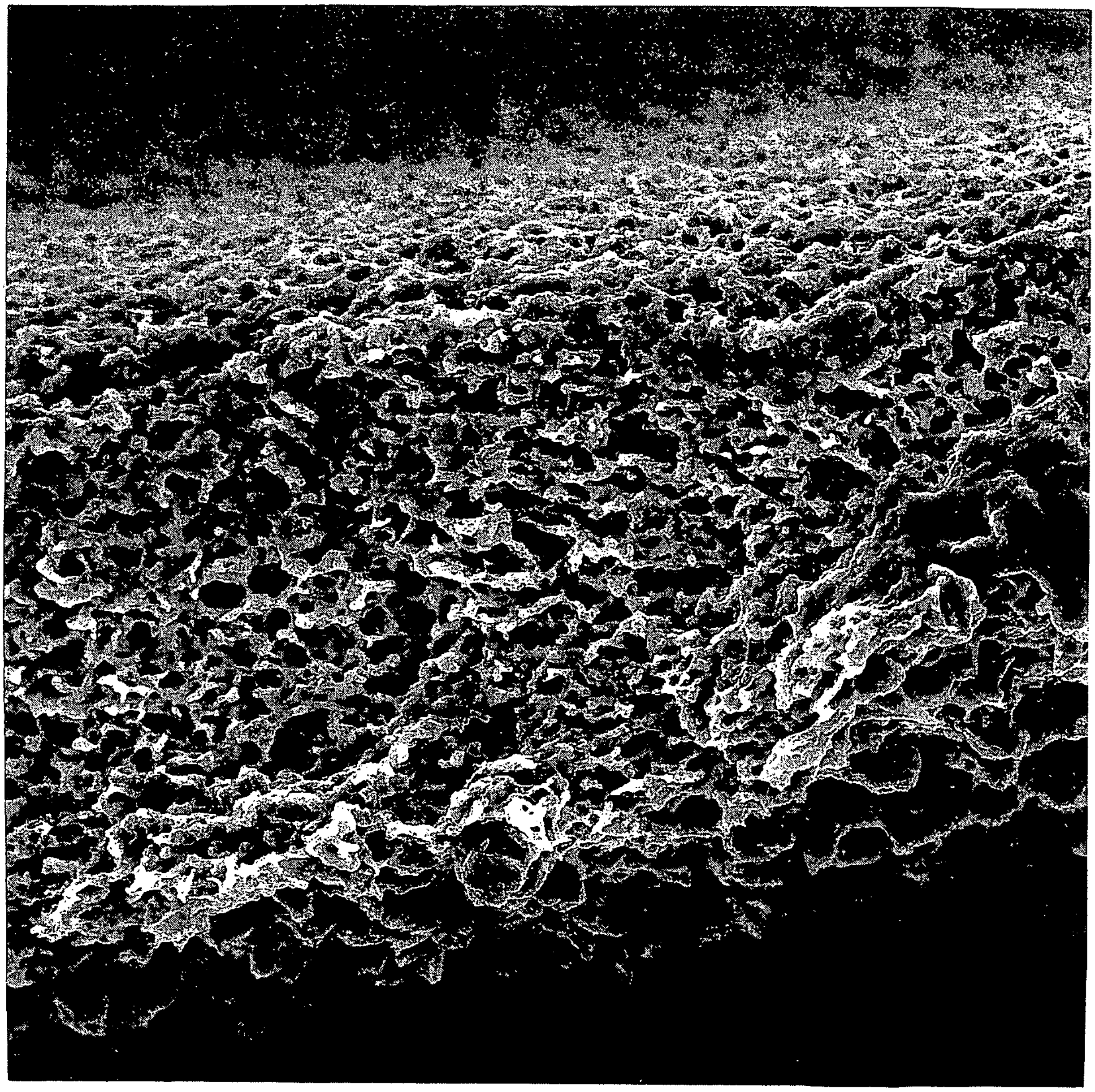

Figure 7

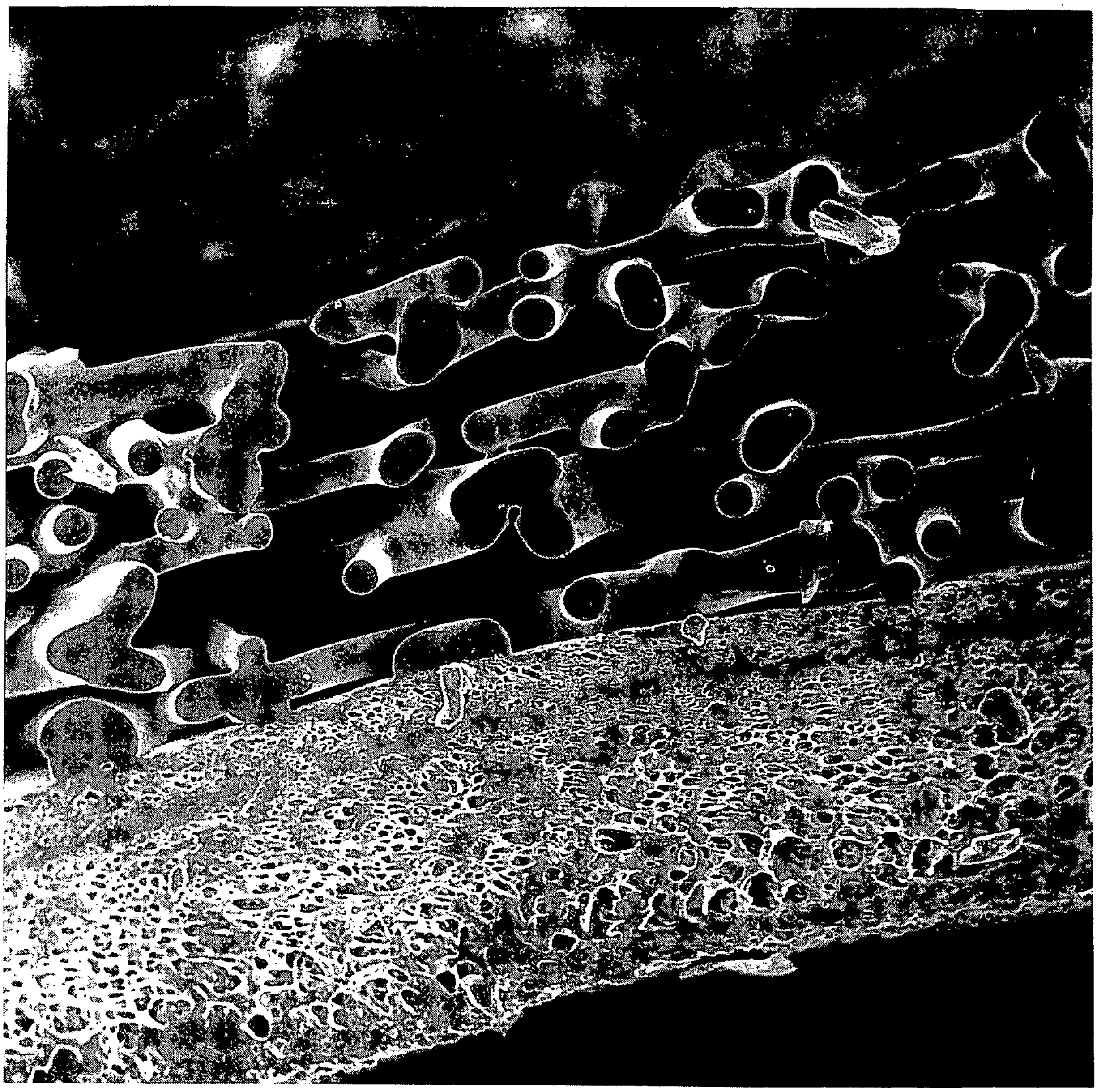

Figure 8

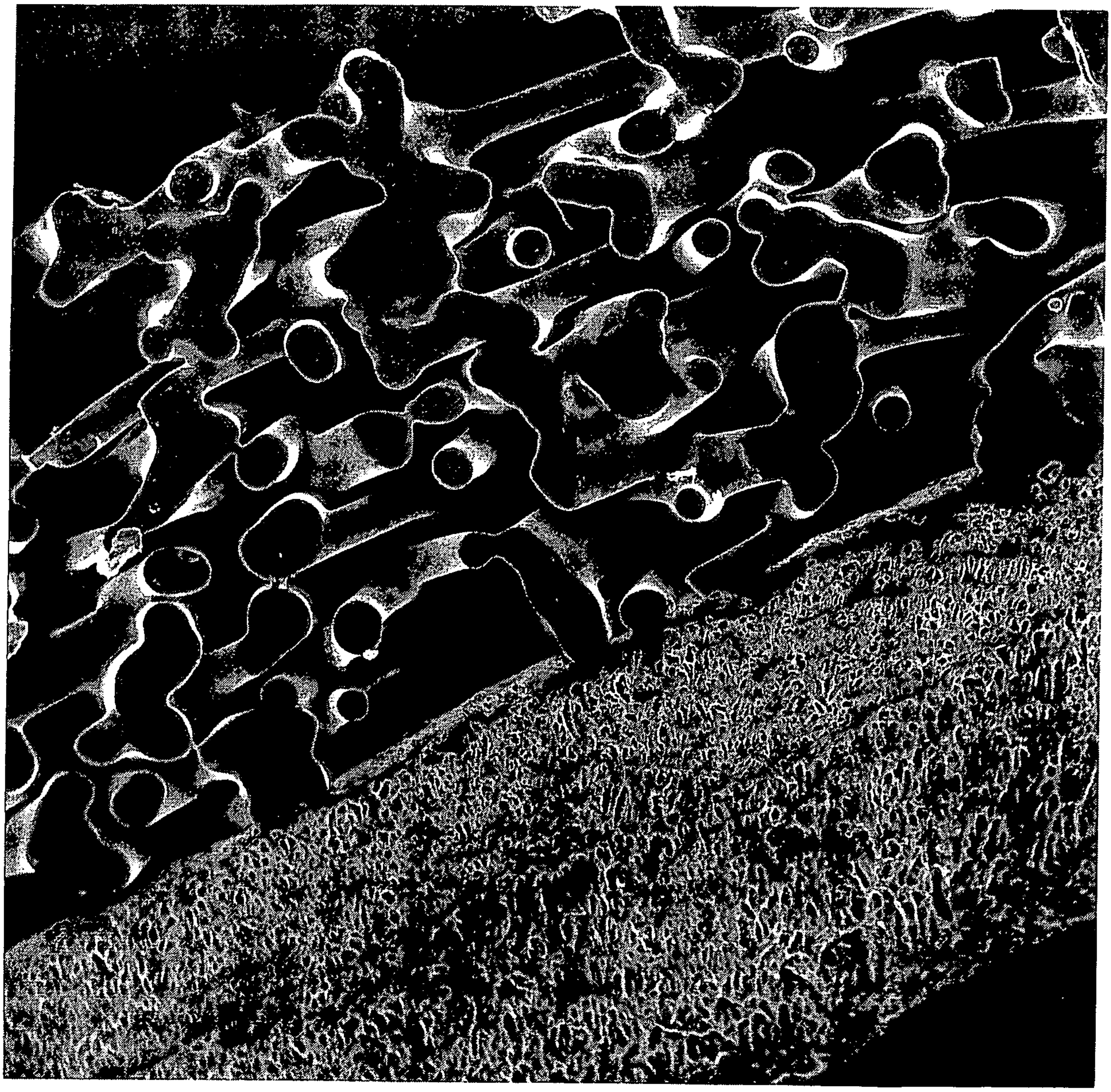

Figure 9

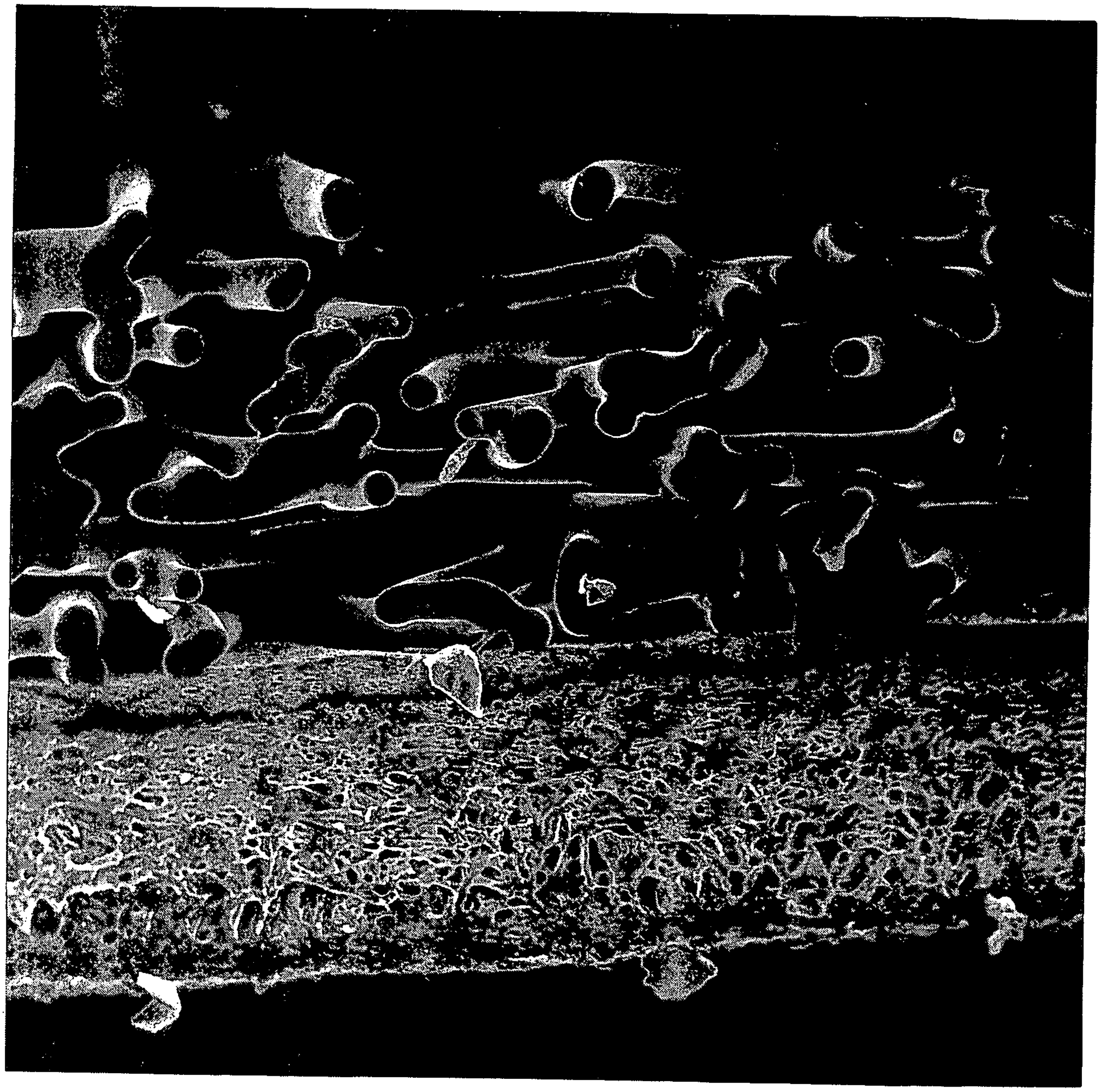

Figure 10

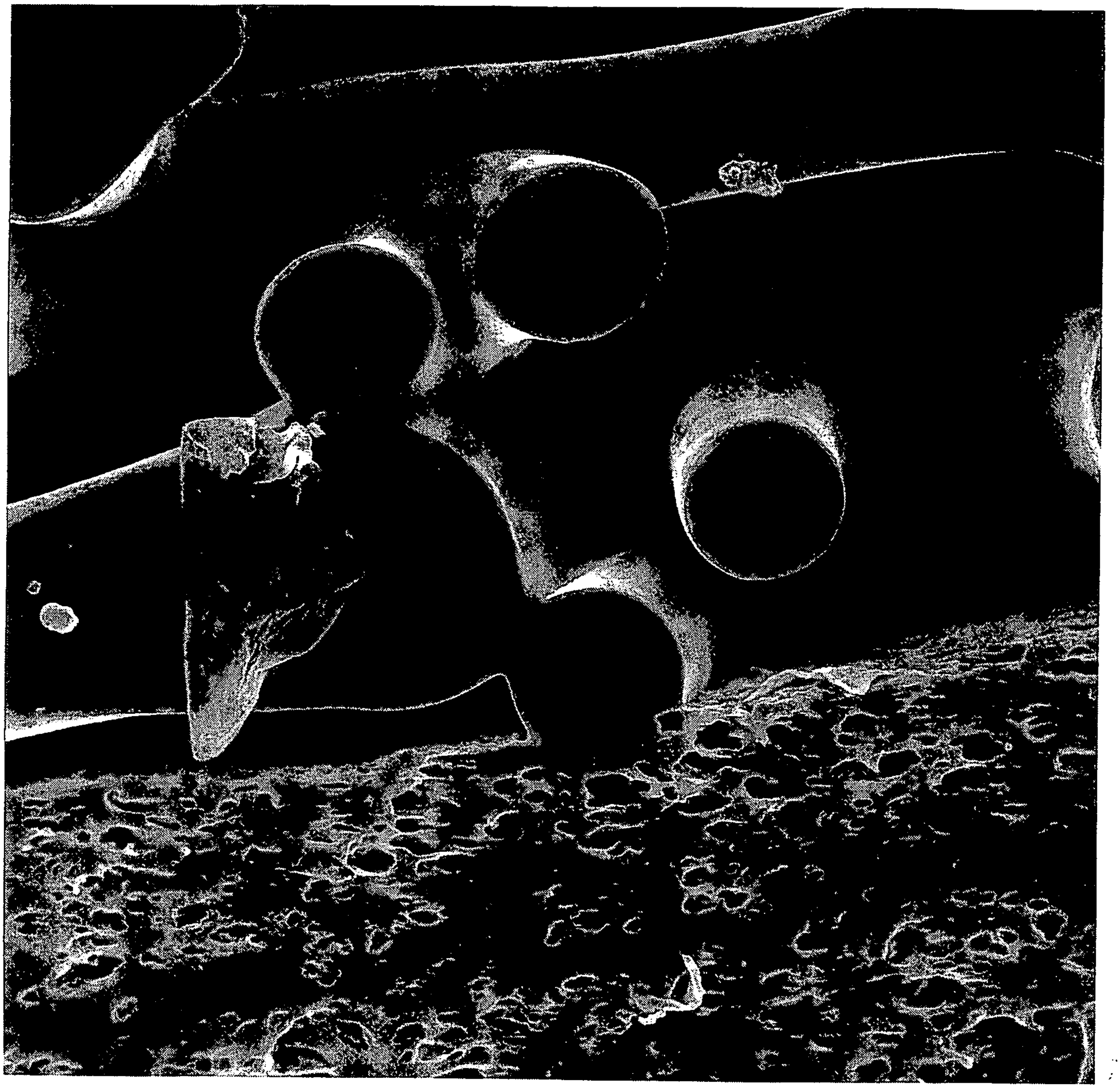

Figure 11

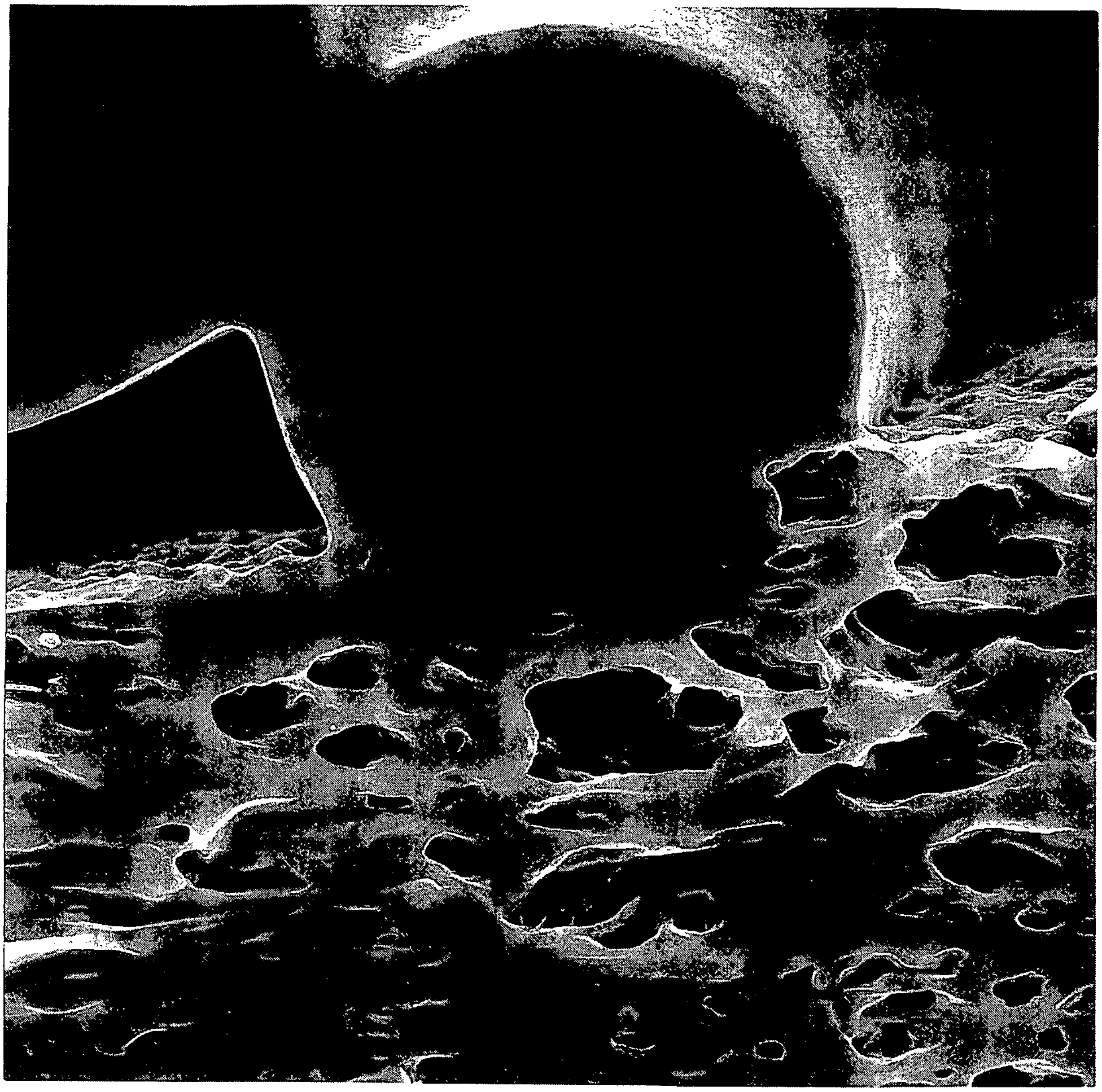

Figure 12

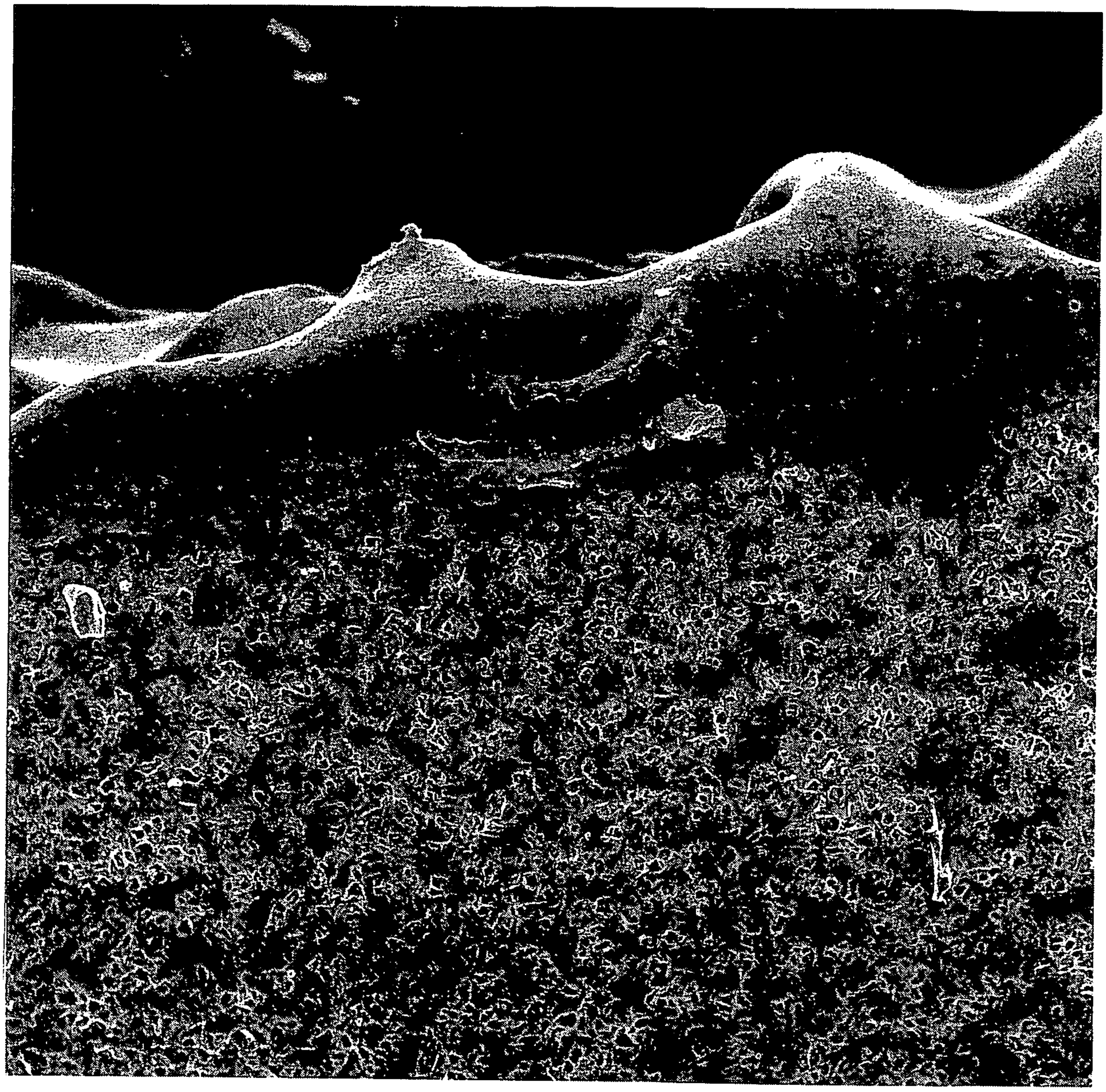

Figure 13

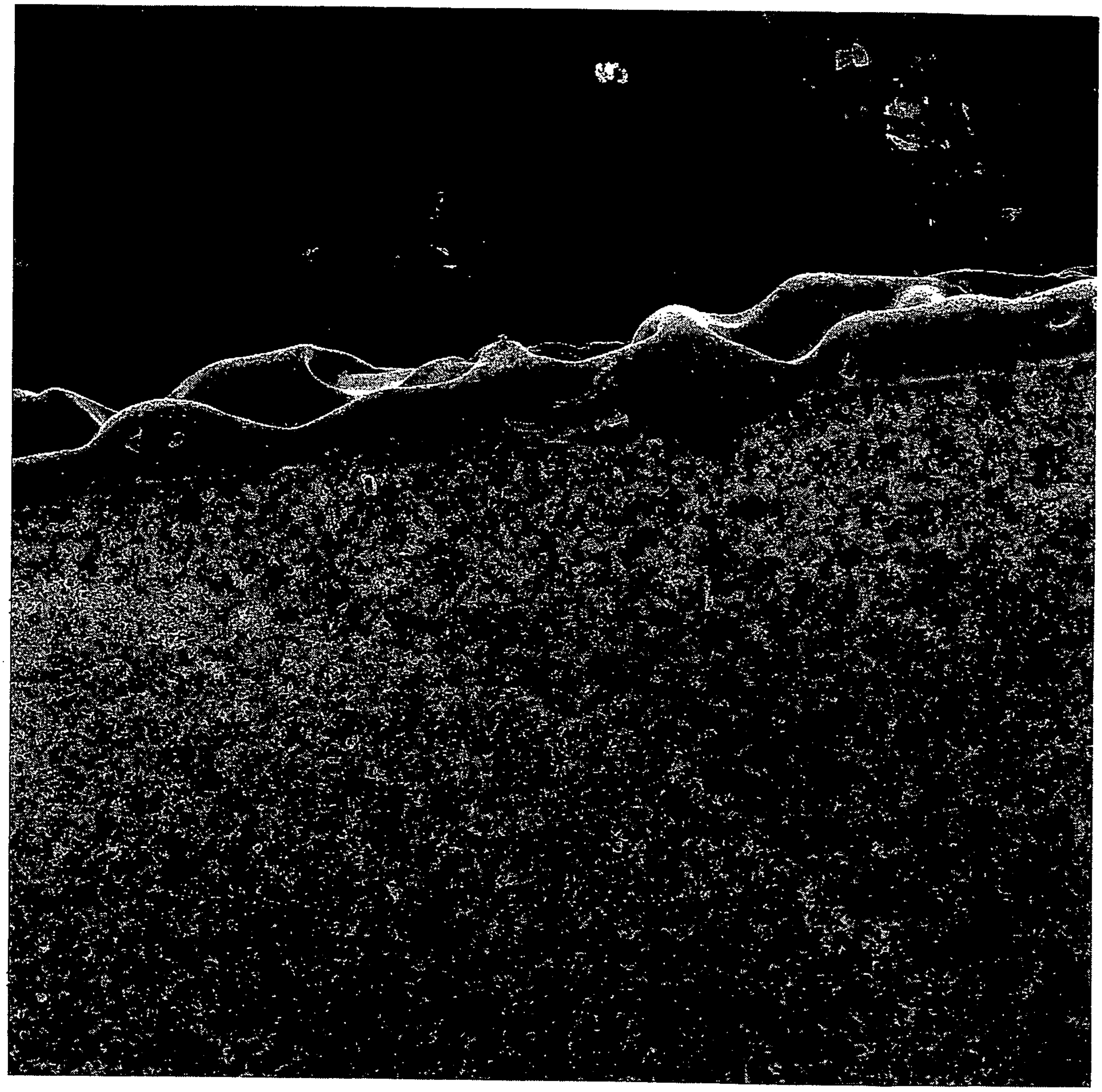

Figure 14

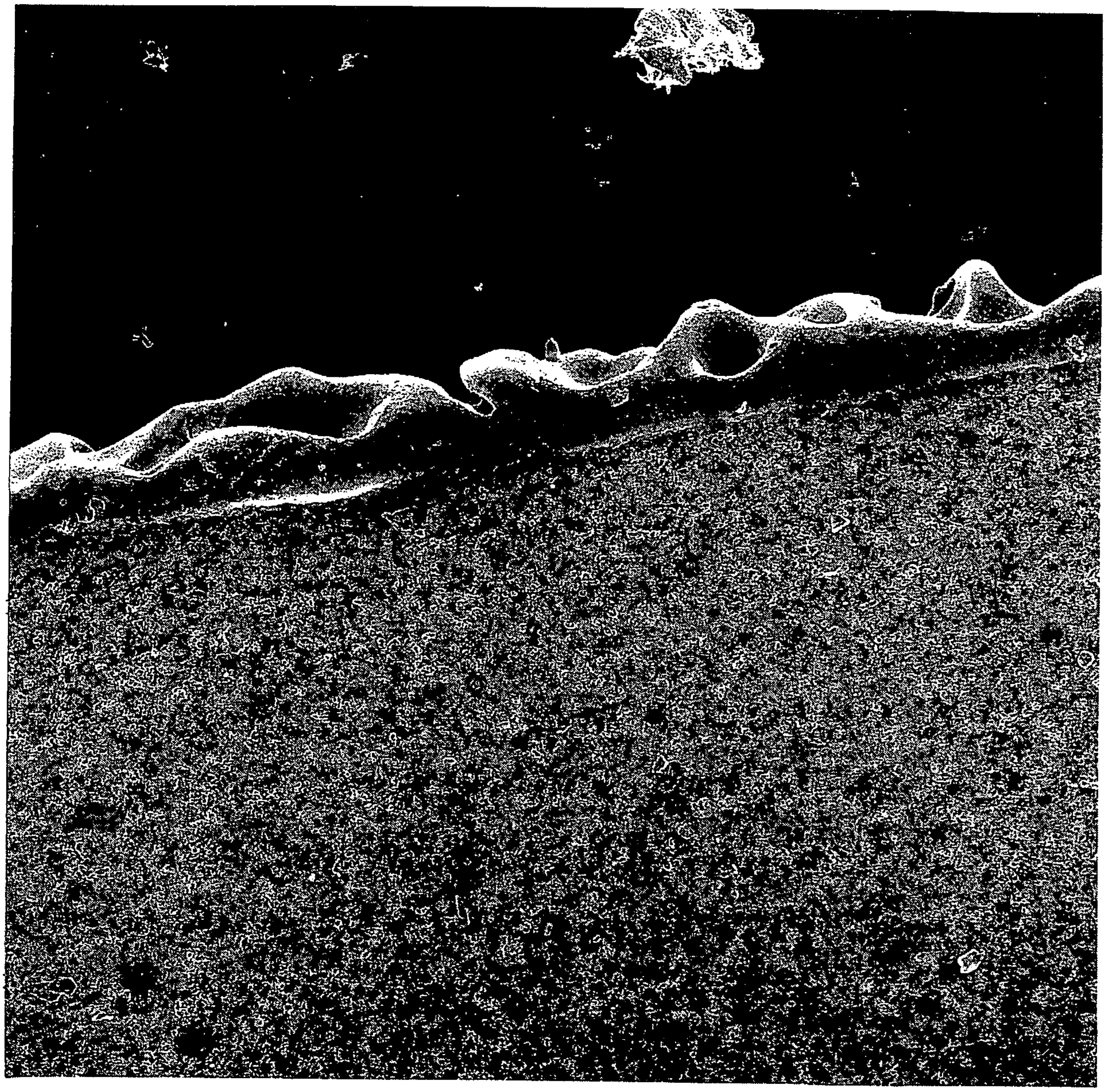

Figure 15

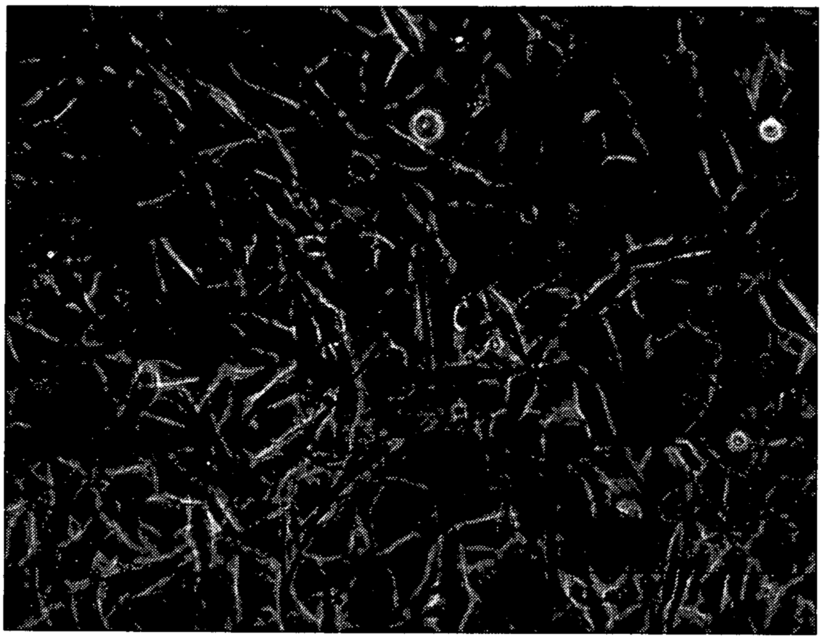
(a)

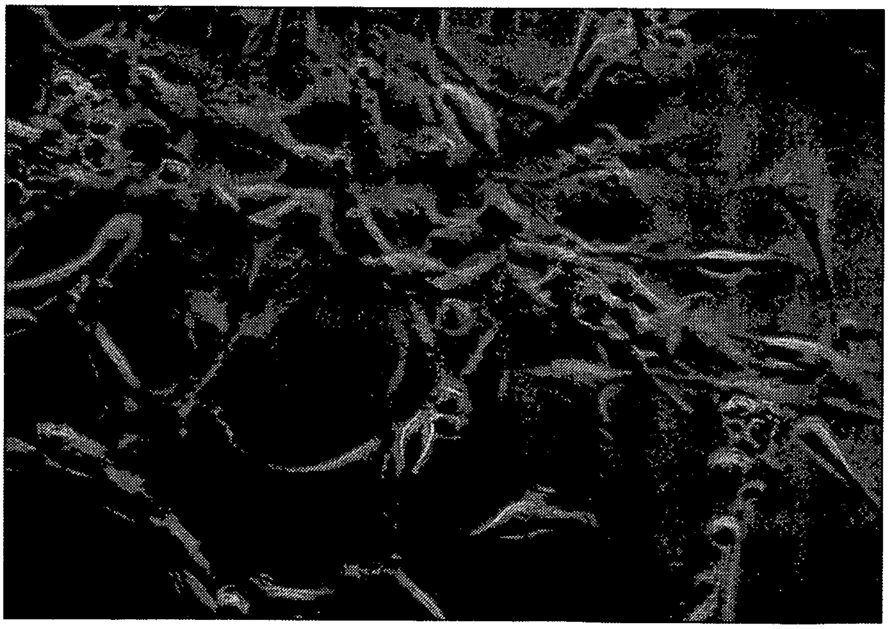
(b)

Figure 16

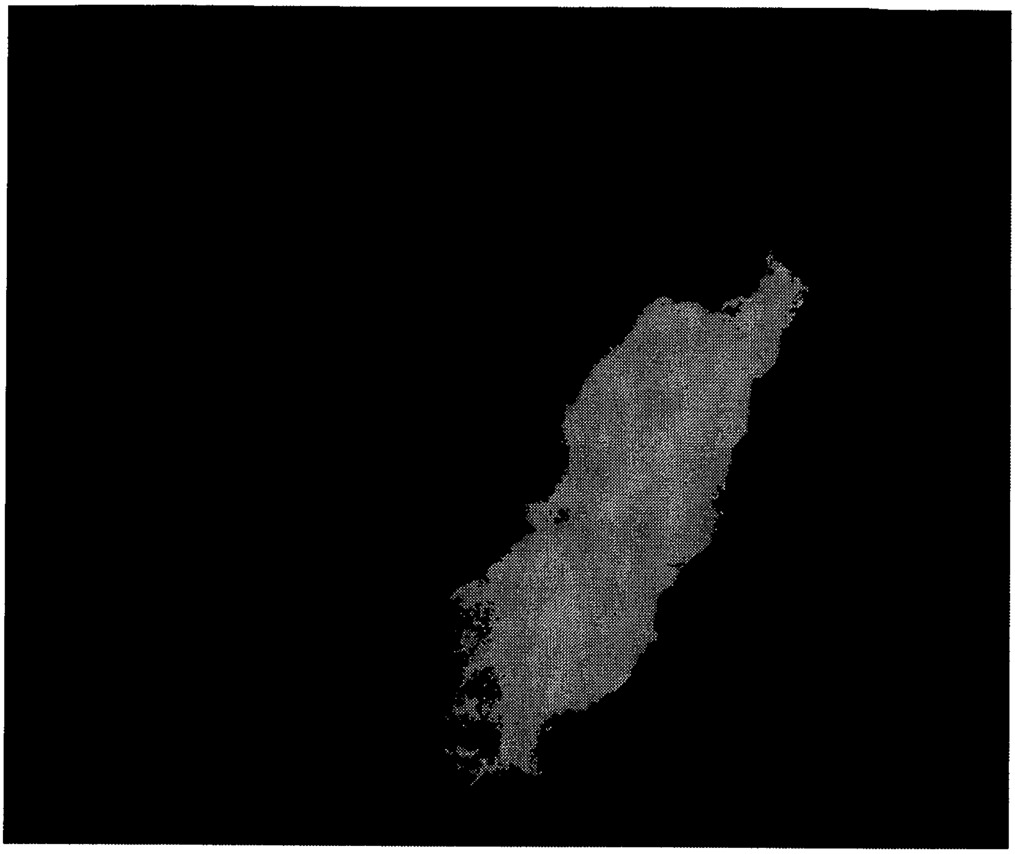

Figure 17

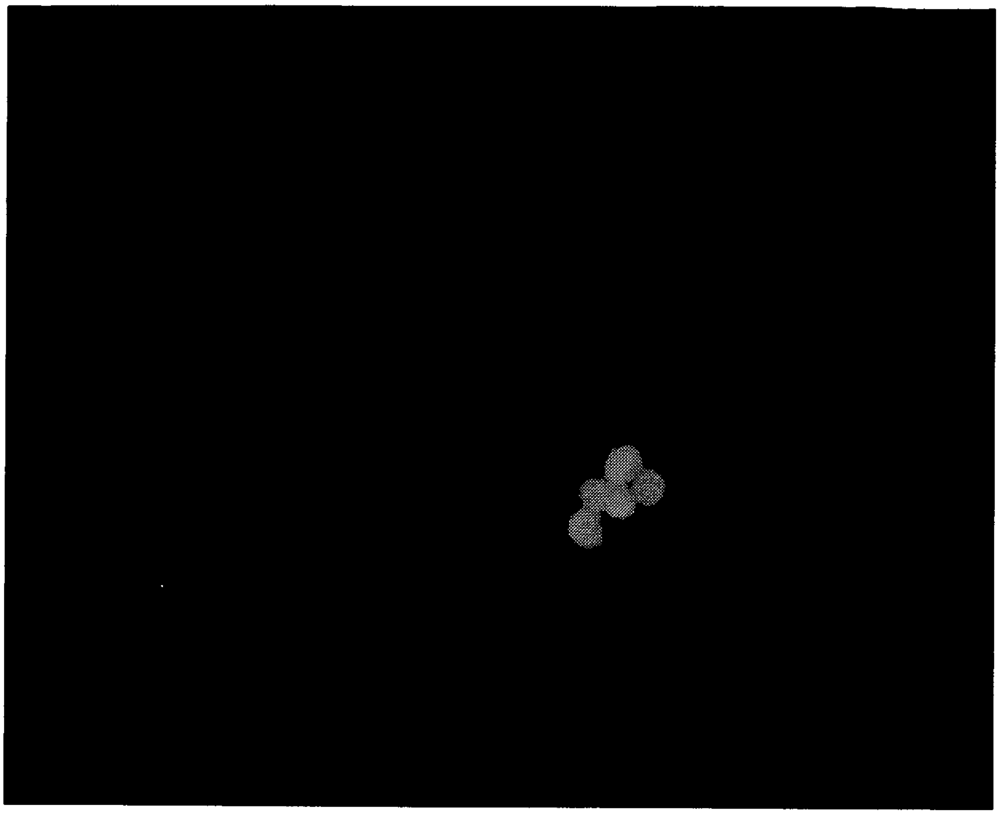

Figure 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0196486 A
- EP 1498147 A
- EP 1452189 A
- EP 1452190 A
- US 20050155926 A
- EP 0696605 A